(19) Europäisches Patentamt
European Patent Office
Office européen des brevets

(11) Veröffentlichungsnummer : **0 475 898 A1**

# (12) EUROPÄISCHE PATENTANMELDUNG

(21) Anmeldenummer : **91810697.2**

(22) Anmeldetag : **03.09.91**

(51) Int. Cl.[5] : **C07D 249/12,** C07D 233/70, C07D 239/22, C07D 243/04, A61K 31/415, A61K 31/41, A61K 31/505

(30) Priorität : **10.09.90 CH 2927/90**

(43) Veröffentlichungstag der Anmeldung : **18.03.92 Patentblatt 92/12**

(84) Benannte Vertragsstaaten : **AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Ostermayer, Franz, Dr.**
**Am Hang 5**
**CH-4125 Riehen (CH)**
Erfinder : **Herold, Peter, Dr.**
**Mattweg 19**
**CH-4144 Arlesheim (CH)**
Erfinder : **Fuhrer, Walter, Dr.**
**Im Budler 3**
**CH-4419 Lupsingen (CH)**

(54) **Azacyclische Verbindungen.**

(57) Azacyclische Verbindungen der Formel

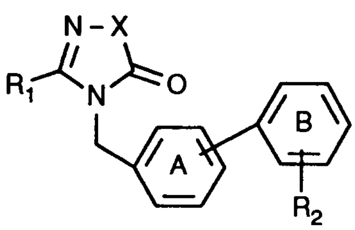

(I),

worin X die Gruppe der Formel $-C(R_3)R_4-[C(R_5)R_6]_p-[C(R_7)R_6]_q-$ (Ia) oder die Gruppe der Formel $-N(R_9)-$ (Ib) ist, $R_1$ unsubstituiertes oder ein- oder mehrfach substituiertes Niederalkyl, Niederalkenyl oder Niederalkinyl, wobei die Substituenten aus der Gruppe ausgewählt sein können, die aus Hydroxy und Halogen besteht, Cycloalkyl, Cycloalkenyl, Phenylniederalkyl, Phenylniederalkenyl oder Phenylniederalkinyl ist, $R_2$ Carboxy, 1H-Tetrazol-5-yl, $SO_3H$, $PO_2H_2$, $PO_3H_2$ oder Halogenalkansulfonylamino ist, entweder $R_3$, $R_4$, $R_5$, $R_6$, $R_7$ und $R_6$ unabhängig voneinander Wasserstoff, unsubstituiertes oder ein-oder mehrfach substituiertes Niederalkyl, Niederalkenyl oder Niederalkinyl, wobei die Substituenten aus der Gruppe ausgewählt sein können, die aus Halogen, gegebenenfalls verethertem Hydroxy, gegebenenfalls verestertem oder amidiertem Carboxy und gegebenenfalls substituiertem Amino besteht, gegebenenfalls verestertes oder amidiertes Carboxy, Cycloalkyl, Cycloalkenyl, einen durch O unterbrochenen aliphatischen Kohlenwasserstoffrest oder einen aromatischen Rest darstellen oder eines der drei Variablenpaare $R_3/R_4$, $R_5/R_6$ und $R_7/R_6$ einen zweiwertigen aliphatischen Kohlenwasserstoffrest darstellt und die Variablen der beiden anderen Variablenpaare unabhängig voneinander die vorstehend angegebenen Bedeutungen haben, $R_9$ Wasserstoff oder unsubstituiertes oder ein- oder mehrfach substituiertes Niederalkyl, Niederalkenyl oder Niederalkinyl, wobei die Substituenten aus der Gruppe ausgewählt sein können, die aus gegebenenfalls verethertem Hydroxy und gegebenenfalls verestertem oder amidiertem Carboxy besteht, ist, die Indices p und q unabhängig voneinander für 0 oder 1 stehen und die Ringe A und B unabhängig voneinander gegebenenfalls substituiert sind, und gegebenenfalls die Tautomeren davon, jeweils in freier Form oder in Sakzform, sind in an sich bekannter Weise herstellbar und können beispielsweise als Arzneimittelwirskstoffe verwendet werden.

EP 0 475 898 A1

Die Erfindung betrifft azacyclische Verbindungen der Formel

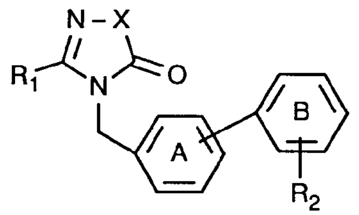

(I),

worin X die Gruppe der Formel $-C(R_3)R_4-[C(R_5)R_6]_p-[C(R_7)R_8]_q-$ (Ia) oder die Gruppe der Formel $-N(R_9)-$ (Ib) ist, $R_1$ unsubstituiertes oder ein- oder mehrfach substituiertes Niederalkyl, Niederalkenyl oder Niederalkinyl, wobei die Substituenten aus der Gruppe ausgewählt sein können, die aus Hydroxy und Halogen besteht, Cycloalkyl, Cycloalkenyl, Phenylniederalkyl, Phenylniederalkenyl oder Phenylniederalkinyl ist, $R_2$ Carboxy, 1H-Tetrazol-5-yl, $SO_3H$, $PO_2H_2$, $PO_3H_2$ oder Halogenalkansulfonylamino ist, entweder $R_3$, $R_4$, $R_5$, $R_6$, $R_7$ und $R_8$ unabhängig voneinander Wasserstoff, unsubstituiertes oder ein-oder mehrfach substituiertes Niederalkyl, Niederalkenyl oder Niederalkinyl, wobei die Substituenten aus der Gruppe ausgewählt sein können, die aus Halogen, gegebenenfalls verethertem Hydroxy, gegebenenfalls verestertem oder amidiertem Carboxy und gegebenenfalls substituiertem Amino besteht, gegebenenfalls verestertes oder amidiertes Carboxy, Cycloalkyl, Cycloalkenyl, einen durch O unterbrochenen aliphatischen Kohlenwasserstoffrest oder einen aromatischen Rest darstellen oder eines der drei Variablenpaare $R_3/R_4$, $R_5/R_6$ und $R_7/R_8$ einen zweiwertigen aliphatischen Kohlenwasserstoffrest darstellt und die Variablen der beiden anderen Variablenpaare unabhängig voneinander die vorstehend angegebenen Bedeutungen haben, $R_9$ Wasserstoff oder unsubstituiertes oder ein- oder mehrfach substituiertes Niederalkyl, Niederalkenyl oder Niederalkinyl, wobei die Substituenten aus der Gruppe ausgewählt sein können, die aus gegebenenfalls verethertem Hydroxy und gegebenenfalls verestertem oder amidiertem Carboxy besteht, ist, die Indices p und q unabhängig voneinander für 0 oder 1 stehen und die Ringe A und B unabhängig voneinander gegebenenfalls substituiert sind, in freier Form oder in Salzform sowie gegebenenfalls die Tautomeren dieser Verbindungen, die Verwendung dieser Verbindungen und Tautomeren, ein Verfahren zur Herstellung dieser Verbindungen und Tautomeren und pharmazeutische Präparate, enthaltend, in freier Form oder in Form eines pharmazeutisch verwendbaren Salzes, eine solche Verbindung I oder ein Tautomeres davon.

Die Verbindungen I können teilweise als Protonentautomere vorliegen. Bedeutet z. B. X eine Gruppe -NH-, können entsprechende, die Gruppierung -NH-C(=O)- enthaltende, Verbindungen I im Gleichgewicht mit den tautomeren Hydroxyderivaten, d. h. den entsprechenden Verbindungen mit einer Gruppierung der Formel -N=C(OH)-, stehen. Entsprechend sind unter den Verbindungen I vor- und nachstehend sinn- und zweckgemäss gegebenenfalls jeweils auch entsprechende Tautomere zu verstehen.

Die Verbindungen I können als, insbesondere pharmazeutisch verwendbare, Salze vorliegen. Weisen die Verbindungen I mindestens ein basisches Zentrum auf, können sie Säureadditionssalze bilden. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie gegebenenfalls, z.B. durch Halogen, substituierten $C_1$ -$C_4$-Alkancarbonsäuren, z.B. Essigsäure, wie gegebenenfalls ungesättigten Dicarbonsäuren, z.B. Oxal-, Malon-, Bernstein-, Malein-, Fumar-, Phthal- oder Terephthalsäure, wie Hydroxycarbonsäuren, z.B. Ascorbin-, Glykol-, Milch-, Äpfel-, Wein- oder Zitronensäure, wie Aminosäuren, z.B. Asparagin- oder Glutaminsäure, oder wie Benzoesäure, oder mit organischen Sulfonsäuren, wie gegebenenfalls, z.B. durch Halogen, substituierten $C_1$-$C_4$-Alkan- oder Aryl-sulfonsäuren, z.B. Methan- oder p-Toluolsulfonsäure, gebildet. Entsprechende Säureadditionssalze können auch mit einem gegebenenfalls zusätzlich vorhandenen basischen Zentrum gebildet werden. Ferner können Verbindungen I mit mindestens einer aciden Gruppe (beispielsweise COOH oder 1H-Tetrazol-5-yl) Salze mit Basen bilden. Geeignete Salze mit Basen sind beispielsweise Metallsalze, wie Alkali- oder Erdalkalimetallsalze, z.B. Natrium-, Kalium- oder Magnesiumsalze, oder Salze mit Ammoniak oder einem organischen Amin, wie Morpholin, Thiomorpholin, Piperidin, Pyrrolidin, einem Mono-, Di- oder Triniederalkylamin, z. B. Ethyl-, tert.-Butyl-, Diethyl-, Diisopropyl-, Triethyl-, Tributyl- oder Dimethyl-propyl-amin, oder einem Mono-, Di- oder Trihydroxynieder-alkylamin, z.B. Mono-, Di- oder Triethanolamin. Weiterhin können entsprechende innere Salze gebildet werden. Umfasst sind ferner für pharmazeutische Verwendungen nicht geeignete Salze, die beispielsweise für die Isolierung bzw. Reinigung von freien Verbindungen I oder deren pharmazeutisch verwendbaren Salzen eingesetzt werden können.

Die Ringe A und B bilden einen Biphenylylrest, wobei entsprechendes 4-Biphenylyl bevorzugt ist und der Rest $R_2$ vorzugsweise in einer 2-Position von Ring B lokalisiert ist. Die Ringe A und B sind unabhängig voneinander unsubstituiert oder ein- oder mehrfach, z.B. zwei- oder dreifach, substituiert, wobei die Substituenten

aus der Gruppe ausgewählt sein können, die aus Halogen, Hydroxy, verethertem Hydroxy, Amino, substituiertem Amino, Carboxy, verestertem Carboxy, amidiertem Carboxy, Niederalkyl, Niederalkenyl, Niederalkinyl, einem durch O unterbrochenen aliphatischen Kohlenwasserstoffrest und Trifluormethyl besteht.

Verethertes Hydroxy ist Niederalkoxy oder Niederalkenyloxy.

Verestertes Carboxy ist Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist.

Amidiertes Carboxy ist Carbamoyl, dessen Aminogruppe unsubstituiert oder durch Niederalkyl, Niederalkenyl, Niederalkinyl, Hydroxyniederalkyl, Niederalkoxyniederalkyl oder Phenyl-niederalkyl, -niederalkenyl oder -niederalkinyl unabhängig voneinander mono- oder disubstituiert oder durch Niederalkylen oder Niederalkylenoxyniederalkylen disubstituiert ist.

Substituiertes Amino ist durch Niederalkyl, Niederalkenyl, Niederalkinyl oder Phenyl-niederalkyl, -niederalkenyl oder -niederalkinyl unabhängig voneinander mono-oder disubstituiertes oder durch Niederalkylen oder Niederalkylenoxyniederalkylen disubstituiertes Amino.

Ein durch O unterbrochener aliphatischer Kohlenwasserstoffrest ist Niederalkoxy-niederalkyl, -niederalkenyl oder -niederalkinyl oder Niederalkenyloxy-niederalkyl, -niederalkenyl oder -niederalkinyl.

Ein aromatischer Rest ist Phenyl, Naphthyl, Pyrrolyl, Pyrazolyl, Imidazolyl, Triazolyl, Tetrazolyl, Furyl, Thienyl oder Pyridyl.

Ein zweiwertiger aliphatischer Kohlenwasserstoffrest ist Niederalkylen oder Niederalkenylen.

Die vorstehend aufgeführten aromatischen Reste sind, ebenso wie die Phenylreste in phenylsubstituierten Gruppen, z. B. in Phenylniederalkylgruppen, unabhängig voneinander unsubstituiert oder ein- oder mehrfach, z.B. zwei- oder dreifach, substituiert, wobei die Substituenten aus der Gruppe ausgewählt sein können, die aus Halogen, Hydroxy, Niederalkoxy, gegebenenfalls substituiertem Amino, gegebenenfalls verestertem oder amidiertem Carboxy, Niederalkyl, Niederalkenyl, Niederalkinyl und Trifluormethyl besteht.

Die vor- und nachstehend verwendeten Allgemeinbegriffe haben, sofern nicht abweichend definiert, die nachfolgend angegebenen Bedeutungen.

Der Ausdruck "Nieder" bedeutet, dass entsprechende Gruppen und Verbindungen jeweils bis und mit 7, vorzugsweise bis und mit 4, Kohlenstoffatome enthalten

Niederalkyl ist $C_1$-$C_7$-Alkyl, d. h. Methyl, Ethyl, n-Propyl, Isopropyl, n-Butyl, Isobutyl, sek.-Butyl, tert.-Butyl oder ein entsprechender Pentyl-, Hexyl- oder Heptylrest. Bevorzugt ist $C_1$-$C_4$-Alkyl.

Niederalkenyl bedeutet $C_3$-$C_7$-Alkenyl und ist z.B.Propen-2-yl, Allyl oder But-1-en-3-yl, -1-en-4-yl, -2-en-1 -yl oder -2-en-2-yl. Bevorzugt ist $C_3$-$C_5$-Alkenyl.

Niederalkinyl ist $C_3$-$C_7$-Alkinyl und bedeutetz.B.Propargyl.

Niederalkylen bedeutet $C_2$-$C_7$-Alkylen, ist geradkettig oder verzweigt und bedeutet insbesondere Ethylen,Prop- 1,3-ylen, But- 1,4-ylen, Pent- 1,5-ylen, Prop-1,2-ylen, 2-Methylprop- 1,3-ylen oder 2,2-Dimethylprop-1,3-ylen. Bevorzugt ist $C_2$-$C_5$-Alkylen.

Niederalkenylen ist $C_3$-$C_5$-Alkenylen und bedeutet z.B. But-2-en- 1,4-ylen.

Cycloalkyl ist $C_3$-$C_7$-Cycloalkyl, d. h. Cyclopropyl, Cyclobutyl, Cyclopentyl, Cyclohexyl oder Cycloheptyl. Bevorzugt sind Cyclopentyl und Cyclohexyl.

Cycloalkenyl ist $C_3$-$C_7$-Cycloalkenyl und bedeutet z. B. Cyclopent-2-enyl oder -3-enyl oder Cyclohex-2-enyl oder -3-enyl.

Phenylniederalkyl ist Phenyl-$C_1$-$C_4$-alkyl und bedeutet z. B. Benzyl oder 1- oder 2-Phenethyl, während Phenylniederalkenyl bzw. Phenylniederalkinyl Phenyl-$C_3$-$C_5$-alkenyl bzw. -alkinyl bedeuten, z. B. 3-Phenylallyl oder 3-Phenylpropargyl.

Hydroxyniederalkyl bedeutet Hydroxy-$C_1$-$C_4$-alkyl, wie Hydroxymethyl, 2-Hydroxyethyl oder 3-Hydroxypropyl.

Niederalkoxy ist $C_1$-$C_7$-Alkoxy d. h. Methoxy, Ethoxy, n-Propyloxy, Isopropyloxy, n-Butyloxy, Isobutyloxy, sek.-Butyloxy, tert.-Butyloxy oder entsprechendes Pentyloxy, Hexyloxy oder Heptyloxy. Bevorzugt ist $C_1$-$C_4$-Alkoxy.

Niederalkenyloxy bedeutet $C_3$-$C_7$-Alkenyloxy und ist z.B. Allyloxy, But-2-en-1-yloxy oder But-3-en-1-yloxy. Bevorzugt ist $C_3$-$C_5$-Alkenyloxy.

Niederalkoxyniederalkyl bedeutet $C_1$-$C_4$-Alkoxy-$C_1$-$C_4$-alkyl, wie 2-Methoxyethyl, 2-Ethoxyethyl, 2-(n-Propyloxy)ethyl oder Ethoxymethyl.

Niederalkoxy-niederalkenyl bzw. -niederalkinyl bedeutet $C_1$ -$C_4$-Alkoxy-$C_3$-$C_5$-alkenyl bzw. -alkinyl.

Niederalkenyloxyniederalkyl bedeutet $C_3$-$C_5$-Alkenyloxy-$C_1$-$C_4$-alkyl, wie 2-Allyloxyethyl, und Niederalkenyloxy-niederalkenyl bzw. -niederalkinyl bedeutet $C_3$-$C_5$-Alkenyloxy-$C_3$-$C_5$-alkenyl bzw.-alkinyl.

Niederalkylenoxyniederalkylen bedeutet $C_2$-$C_4$-Alkylenoxy-$C_2$-$C_4$-alkylen, z. B. Ethylen-oxyethylen.

Halogen ist insbesondere Halogen mit einer Atomnummer bis und mit 35, d. h. Fluor, Chlor oder Brom,

und umfasst ferner Iod.

Halogenalkansulfonylamino bedeuted Halogen $C_1$-$C_7$-alkansylfonylamino und ist z.B. Trifluormethan-, Difluormethan-, 1,1,2-Trifluorethan-oder Heptafluorpropan- sulfonylamino. Bevorzugt ist Halogen-$C_1$-$C_4$-alkansulfonylamino.

Ausgedehnte pharmakologische Untersuchungen haben ergeben, dass die Verbindungen I und ihre pharmazeutisch verwendbaren Salze z. B. ausgeprägte Angiotensin-II-antagonisierende Eigenschaften aufweisen.

Bekanntlich hat Angiotensin-II starke vasokonstriktorische Eigenschaften und stimuliert ausserdem die Aldosteronsekretion und bewirkt somit eine deutliche Natrium/Wasser-Retention. Die Folge der Angiotensin-II-Aktivität manifestiert sich unter anderem in einer Erhöhung des Blutdrucks.

Die Bedeutung von Angiotensin-II-Antagonisten besteht darin, durch kompetitive Hemmung der Bindung von Angiotensin-II an die Rezeptoren die durch Angiotensin-II bewirkten vasokonstriktorischen und die Aldosteronsekretion-stimulierenden Effekte zu unterdrücken.

Die Angiotensin-II-antagonisierenden Eigenschaften der Verbindungen I und ihrer pharmazeutisch verwendbaren Salze können im Angiotensin-II-Bindungstest erfasst werden. Dabei werden glatte Muskelzellen der Ratte aus homogenisierter Rattenaorta verwendet. Das feste Zentrifugat wird in 50 mM Tris-Puffer (pH 7.4) unter Einsatz von Peptidaseinhibitoren suspendiert. Die Proben werden 60 Minuten bei 25°C mit $^{125}$I-Angiotensin-II (0.175 nM) und einer variierenden Konzentration an Angiotensin-II oder an Testsubstanz inkubiert. Die Inkubation wird dann durch Zugabe von mit eiskaltem Phosphat gepuffertem Kochsalz beendet und es wird durch Whatman GF/F Filter filtriert. Die Filter werden mit einem Gamma-Zähler gezählt. Aus der Dosis-Wirkungs-Kurve werden die $IC_{50}$-Werte bestimmt. Für die Verbindungen I und ihre pharmazeutisch verwendbaren Salze werden $IC_{50}$-Werte ab etwa 10 nM ermittelt.

Zur Bestimmung der Angiotensin-II induzierten Vasokonstriktion können Untersuchungen an dem isolierten Kaninchen-Aortaring herangezogen werden. Hierzu werden von jeder Brust Aortaringe präpariert und zwischen 2 parallelen Klammem bei einer anfänglich bestehenden Spannung von 2 g fixiert. Anschliessend werden die Ringe bei 37°C in 20 ml eines Gewebebades getaucht und mit einem Gemisch aus 95 % $O_2$ und 5 % $CO_2$ begast. Die isometrischen Reaktionen werden gemessen. In 20-minütigen Intervallen werden die Ringe abwechselnd mit 10 nM Angiotensin-II (Hypertensin-CIBA) und 5 nM Noradrenalinchlorid stimuliert. Anschliessend werden die Ringe mit ausgewählten Konzentrationen der Testsubstanzen vor der Behandlung mit den Agonisten inkubiert. Die Daten werden mit einem Buxco Digitalcomputer analysiert. Die Konzentrationen, die eine 50%-ige Hemmung der Anfangskontrollwerte bewirken, werden als $IC_{50}$-Werte angegeben. Für die Verbindungen I und ihre pharmazeutisch verwendbaren Salze werden $IC_{50}$-Werte ab etwa 5 nM bestimmt.

Dass die Verbindungen I und ihre pharmazeutisch verwendbaren Salze durch Angiotensin-II induzierten Bluthochdruck reduzieren können, kann im Testmodell der normotensiven, narkotisierten Ratte verifiziert werden. Nach Kalibration der Präparationen mit jeweils 0.9 % NaCl (1 ml/kg i.v.), Noradrenalin (1 µg/kg i.v.) bzw. Angiotensin-II (0.3 µg/kg i.v.) werden steigende Dosen (3-6) der Testsubstanz durch Bolusinjektion intravenös injiziert, worauf nach jeder Dosis in 5 Minuten-Intervallen Angiotensin-II bzw. Noradrenalin appliziert wird. Der Blutdruck wird direkt in der Halsschlagader gemessen und mit einem on-line Datenerfassungssystem aufgezeichnet (Buxco). Die Spezifität des Angiotensin-II-Antagonismus wird angezeigt durch die selektive Hemmung des von Angiotensin-II, nicht aber des durch Noradrenalin hervorgerufenen Druckeffektes. In diesem Testmodell zeigen die Verbindungen I und ihre pharmazeutisch verwendbaren Salze ab einer Dosis von etwa 0.3 mg/kg i.v. einen hemmenden Effekt.

Auch im Testmodell der renalen hypertensiven Ratte kann die antihypertensive Aktivität der Verbindungen I und ihrer pharmazeutisch verwendbaren Salze manifestiert werden. Bei männlichen Ratten wird durch Verengung einer renalen Arterie gemäss der Goldblatt-Methode Bluthochdruck erzeugt. Den Ratten werden mittels einer Magensonde Dosen der Testsubstanz verabreicht. Kontrolltiere erhalten ein äquivalentes Volumen an Lösungsmittel. Blutdruck und Herzschlag werden indirekt an wachen Tieren nach der Schwanzklemm-Methode von Gerold et al. [Helv. Physiol. Acta 24 (1966), 58] vor Verabreichung er Testsubstanz bzw. des Lösungsmittels sowie während des Verlaufs der Experimente in Intervallen gemessen. Der ausgeprägte antihypertensive Effekt kann ab einer Dosis von etwa 30 mg/kg p.o. nachgewiesen werden.

Dementsprechend können die Verbindungen I und ihre pharmazeutisch verwendbaren Salze z.B. als Wirkstoffe in Antihypertensiva verwendet werden, welche z.B. zur Behandlung von Bluthochdruck sowie von Herzinsuffizienz eingesetzt werden. Ein Erfindungsgegenstand ist somit die Verwendung der Verbindungen I und ihrer pharmazeutisch verwendbaren Salze zur Herstellung von entsprechenden Arzneimitteln und zur therapeutischen Behandlung von Bluthochdruck sowie von Herzinsuffizienz. Bei der Herstellung der Arzneimittel ist auch die gewerbsmässige Herrichtung der Wirksubstanzen eingeschlossen.

Die Erfindung betrifft in erster Linie eine Verbindung der Formel I, worin X die Gruppe Ia oder die Gruppe Ib ist, $R_1$ unsubstituiertes oder ein- oder mehrfach substituiertes Niederalkyl, Niederalkenyl oder Niederalkinyl, wobei die Substituenten aus der Gruppe ausgewählt sein können, die aus Hydroxy und Halogen besteht, Cycloalkyl, Cycloalkenyl, Phenylniederalkyl, Phenylniederalkenyl oder Phenylniederalkinyl ist, $R_2$ Carboxy, 1H-Tetrazol-5-yl, $SO_3H$, $PO_2H_2$, $PO_3H_2$ oder Halogenalkansulfonylamino ist, entweder $R_3$, $R_4$, $R_5$, $R_6$, $R_7$ und $R_6$ unabhängig voneinander Wasserstoff, unsubstituiertes oder ein-oder mehrfach substituiertes Niederalkyl, Niederalkenyl oder Niederalkinyl, wobei die Substituenten aus der Gruppe ausgewählt sein können, die aus Halogen, Hydroxy, Niederalkoxy, Niederalkenyloxy, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl, Hydroxyniederalkyl, Niederalkoxyniederalkyl oder Phenyl-niederalkyl, -niederalkenyl oder -niederalkinyl unabhängig voneinander mono-oder disubstituiert oder durch Niederalkylen oder Niederalkylenoxyniederalkylen disubstituiert ist, Amino und durch Niederalkyl, Niederalkenyl, Niederalkinyl oder Phenyl-niederalkyl, -niederalkenyl oder -niederalkinyl unabhängig voneinander mono-oder disubstituiertem oder durch Niederalkylen oder Niederalkylenoxyniederalkylen disubstituiertem Amino besteht, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl, Hydroxyniederalkyl, Niederalkoxyniederalkyl oder Phenyl-niederalkyl, -niederalkenyl oder -niederalkinyl unabhängig voneinander mono- oder disubstituiert oder durch Niederalkylen oder Niederalkylenoxyniederalkylen disubstituiert ist, Cycloalkyl, Cycloalkenyl, Niederalkoxy-niederalkyl, -niederalkenyl oder -niederalkinyl, Niederalkenyloxy-niederalkyl, -niederalkenyl oder -niederalkinyl, Phenyl, Naphthyl, Pyrrolyl, Pyrazolyl, Imidazolyl, Triazolyl, Tetrazolyl, Furyl, Thienyl oder Pyridyl darstellen oder eines der drei Variablenpaare $R_3/R_4$, $R_6/R_6$ und $R_7/R_8$ Niederalkylen oder Niederalkenylen darstellt und die Variablen der beiden anderen Variablenpaare unabhängig voneinander die unmittelbar vorstehend angegebenen Bedeutungen haben, $R_9$ Wasserstoff oder unsubstituiertes oder ein- oder mehrfach substituiertes Niederalkyl, Niederalkenyl oder Niederalkinyl, wobei die Substituenten aus der Gruppe ausgewählt sein können, die aus Niederalkoxy, Niederalkenyloxy, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -nieder-alkinyl gebunden ist, und Carbamoyl, dessen Aminogruppe unsubstituiert oder durch Niederalkyl, Niederalkenyl, Niederalkinyl, Hydroxyniederalkyl, Niederalkoxyniederalkyl oder Phenyl-niederalkyl, -niederalkenyl oder -niederalkinyl unabhängig voneinander mono- oder disubstituiert oder durch Niederalkylen oder Niederalkylenoxyniederalkylen disubstituiert ist, besteht, ist, die Indices p und q unabhängig voneinander für 0 oder 1 stehen und die Ringe A und B unabhängig voneinander unsubstituiert oder ein- oder mehrfach substituiert sind, wobei die Substituenten aus der Gruppe ausgewählt sein können, die aus Halogen, Hydroxy, Niederalkoxy, Niederalkenyloxy, Amino, durch Niederalkyl, Niederalkenyl, Niederalkinyl oder Phenyl-niederalkyl, -niederalkenyl oder -niederalkinyl unabhängig voneinander mono- oder disubstituiertem oder durch Niederalkylen oder Niederalkylenoxyniederalkylen disubstituiertem Amino, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl, Hydroxyniederalkyl, Niederalkoxyniederalkyl oder Phenyl-niederalkyl, -niederalkenyl oder -niederalkinyl unabhängig voneinander mono- oder disubstituiert oder durch Niederalkylen oder Niederalkylenoxyniederalkylen disubstituiert ist, Niederalkyl, Niederalkenyl, Niederalkinyl, Niederalkoxy-niederalkyl, -niederalkenyl und -niederalkinyl, Niederalkenyl-oxy-niederalkyl, -niederalkenyl und -niederalkinyl und Trifluormethyl besteht, oder gegebenenfalls ein Tautomeres davon, jeweils in freier Form oder in Salzform.

Die Erfindung betrifft in erster Linie eine Verbindung der Formel I, worin X die Gruppe Ia oder die Gruppe Ib ist, $R_1$ Niederalkyl, Niederalkenyl, Niederalkinyl, Cycloalkyl oder Phenylniederalkyl ist, $R_2$ Carboxy, 1H-Tetrazol-5-yl, $SO_3H$, $PO_2H_2$, $PO_3H_2$ oder Halogenalkansulfonylamino ist, entweder $R_3$, $R_4$, $R_5$, $R_6$, $R_7$ und $R_6$ unabhängig voneinander Wasserstoff, Niederalkyl, Niederalkenyl, Niederalkinyl, Carboxy, Carboxy, welches durch einen Alkohol verestert ist, dessen alkoholische Hydroxyfunktion an Niederalkyl gebunden ist, Carbamoyl, dessen Aminogruppe unsubstituiert oder durch Niederalkyl unabhängig voneinander mono- oder disubstituiert ist, Cycloalkyl, Niederalkoxy-niederalkyl, Phenyl, Naphthyl, Pyrrolyl, Pyrazolyl, Imidazolyl, Triazolyl, Tetrazolyl, Furyl, Thienyl oder Pyridyl darstellen oder eines der drei Variablenpaare $R_3/R_4$, $R_5/R_6$ und $R_7/R_8$ Niederalkylen oder Niederalkenylen darstellt und die Variablen der beiden anderen Variablenpaare unabhängig voneinander die unmittelbar vorstehend angegebenen Bedeutungen haben, $R_9$ Wasserstoff, Niederalkyl, Niederalkenyl oder Niederalkinyl ist, entweder die Indices p und q für 0 stehen oder einer der Indices p und q 0 ist und der andere der Indices p und q 1 ist, die Ringe A und B unabhängig voneinander unsubstituiert oder ein- oder mehrfach substituiert sind, wobei die Substituenten aus der Gruppe ausgewählt sein können, die aus Halogen, Hydroxy, Niederalkoxy, Amino, durch Niederalkyl oder Phenylniederalkyl unabhängig voneinander mono- oder disubstituiertem Amino, Carboxy, Carboxy, welches durch einen Alkohol verestert ist, dessen alkoholische Hydroxyfunktion an Niederalkyl gebunden ist, Carbamoyl, dessen Aminogruppe unsubstituiert oder durch Niederalkyl

oder Phenylniederalkyl unabhängig voneinander mono- oder disubstituiert oder durch Niederalkylen oder Niederalkylenoxyniederalkylen disubstituiert ist, Niederalkyl und Trifluormethyl besteht, und der Ring B in 4-Position des Ringes A und $R_2$ in einer 2-Position des Ringes B gebunden ist, oder gegebenenfalls ein Tautomeres davon, jeweils in freier Form oder in Salzform.

Die Erfindung betrifft insbesondere eine Verbindung der Formel I, worin X die Gruppe Ia oder die Gruppe Ib ist, $R_1$ $C_1$-$C_4$-Alkyl, wie n-Propyl oder n-Butyl, ist, $R_2$ Carboxy oder 1H-Tetrazol-5-yl ist, entweder $R_3$, $R_4$, $R_5$, $R_6$, $R_7$ und $R_6$ unabhängig voneinander Wasserstoff oder $C_1$-$C_4$-Alkyl, wie Methyl oder Ethyl, darstellen oder eines der drei Variablenpaare $R_3/R_4$, $R_5/R_6$ und $R_7/R_8$ $C_2$-$C_5$-Alkylen, wie But-1,4-ylen oder Pent- 1,5-ylen, darstellt und die Variablen der beiden anderen Variablenpaare unabhängig voneinander die unmittelbar vorstehend angegebenen Bedeutungen haben, $R_9$ Wasserstoff oder $C_1$-$C_4$-Alkyl, wie Methyl, ist, entweder die Indices p und q für 0 stehen oder einer der Indices p und q 0 ist und der andere der Indices p und q 1 ist, die Ringe A und B unsubstituiert sind und der Ring B in 4-Position des Ringes A und $R_2$ in einer 2-Position des Ringes B gebunden ist, oder gegebenenfalls ein Tautomeres davon, jeweils in freier Form oder in Salzform.

Die Erfindung betrifft vor allem eine Verbindung der Formel I, worin X die Gruppe Ia oder die Gruppe Ib ist, $R_1$ $C_1$-$C_4$-Alkyl, wie n-Propyl oder n-Butyl, ist, $R_2$ 1H-Tetrazol-5-yl ist, entweder $R_3$, $R_4$, $R_5$, $R_6$, $R_7$ und $R_6$ unabhängig voneinander Wasserstoff oder $C_1$-$C_4$-Alkyl, wie Methyl oder Ethyl, darstellen oder eines der drei Variablenpaare $R_3/R_4$, $R_6R_6$ und $R_7/R_8$ $C_2$-$C_5$-Alkylen, wie But-1,4-ylen oder Pent- 1,5-ylen, darstellt und die Variablen der beiden anderen Variablenpaare Wasserstoff bedeuten, $R_9$ Wasserstoff ist, entweder die Indices p und q für 0 stehen oder einer der Indices p und q 0 ist und der andere der Indices p und q 1 ist, die Ringe A und B unsubstituiert sind und der Ring B in 4-Position des Ringes A und $R_2$ in einer 2-Position des Ringes B gebunden ist, oder gegebenenfalls ein Tautomeres davon, jeweils in freier Form oder in Salzform.

Die Erfindung betrifft insbesondere eine Verbindung der Formel I, worin X die Gruppe Ia ist, $R_1$ $C_1$-$C_4$-Alkyl, wie n-Propyl oder n-Butyl, ist, $R_2$ 1H-Tetrazol-5-yl ist, entweder $R_3$, $R_4$, $R_6$, $R_6$, $R_7$ und $R_8$ unabhängig voneinander Wasserstoff oder $C_1$-$C_4$-Alkyl, wie Methyl oder Ethyl, darstellen oder eines der drei Variablenpaare $R_3/R_4$, $R_5/R_6$ und $R_7/R_6$ $C_2$-$C_5$-Alkylen, wie But-1,4-ylen oder Pent-1,5-ylen, darstellt und die Variablen der beiden anderen Variablenpaare Wasserstoff bedeuten, entweder die Indices p und q für 0 stehen oder einer der Indices p und q 0 ist und der andere der Indices p und q 1 ist, die Ringe A und B unsubstituiert sind und der Ring B in 4-Position des Ringes A und $R_2$ in einer 2-Position des Ringes B gebunden ist, in freier Form oder in Salzform.

Die Erfindung betrifft in allererster Linie eine Verbindung der Formel I, worin X die Gruppe Ia ist, $R_1$ $C_1$-$C_4$-Alkyl, wie n-Propyl oder n-Butyl, ist, $R_2$ 1H-Tetrazol-5-yl ist, $R_3$, $R_4$, $R_6$, $R_6$, $R_7$ und $R_8$ unabhängig voneinander Wasserstoff oder $C_1$-$C_4$-Alkyl, wie Methyl oder Ethyl, darstellen, entweder die Indices p und q für 0 stehen oder einer der Indices p und q 0 ist und der andere der Indices p und q 1 ist, die Ringe A und B unsubstituiert sind und der Ring B in 4-Position des Ringes A und $R_2$ in einer 2-Position des Ringes B gebunden ist, in freier Form oder in Salzform.

Die Erfindung betrifft namentlich die in den Beispielen genannten neuen Verbindungen der Formel I sowie gegebenenfalls Tautomere davon, jeweils in freier Form oder in Salzform.

Ein weiterer Gegenstand der Erfindung ist ein Verfahren zur Herstellung der Verbindungen I und ihrer Salze, z.B. dadurch gekennzeichnet, dass man

a) in einer Verbindung der Formel

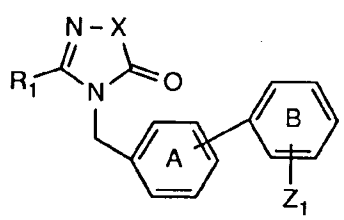

(II),

worin $Z_1$ einen in $R_2$ überführbaren Rest darstellt, oder in einem Salz davon $Z_1$ in $R_2$ überführt oder

b) eine Verbindung der Formel

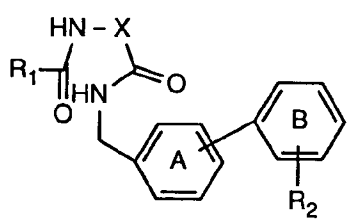

(III)

oder ein Salz davon cyclisiert oder

c) eine Verbindung der Formel

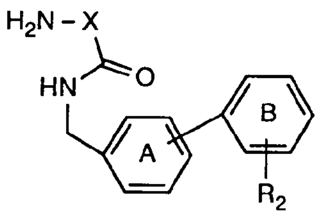

(IV)

oder ein Salz davon mit einer Verbindung der Formel

$$R_1\text{-}C(Z_2)(Z_3)Z_4 \quad (V),$$

worin entweder $Z_2$ und $Z_3$ gemeinsam gegebenenfalls funktionell abgewandeltes Oxo bedeuten und $Z_4$ eine nucleofuge Abgangsgruppe ist oder $Z_2$, $Z_3$ und $Z_4$ unabhängig voneinander für eine nucleofuge Abgangsgruppe stehen, umsetzt und jeweils, wenn erwünscht, eine verfahrensgemäss oder auf andere Weise erhältliche Verbindung I oder ein Tautomeres davon, jeweils in freier Form oder in Salzform, in eine andere Verbindung I oder ein Tautomeres davon überführt, ein verfahrensgemäss erhältliches Gemisch von Isomeren auftrennt und das gewünschte Isomere isoliert und/oder eine verfahrensgemäss erhältliche freie Verbindung I oder ein Tautomeres davon in ein Salz oder ein verfahrensgemäss erhältliches Salz einer Verbindung I oder eines Tautomeren davon in die freie Verbindung I oder ein Tautomeres davon oder in ein anderes Salz überführt.

Salze von Ausgangsmaterialien, die mindestens ein basisches Zentrum aufweisen, sind entsprechende Säureadditionssalze, während Salze von Ausgangsstoffen, die mindestens eine acide Gruppe aufweisen, Salze mit Basen sind, jeweils wie in Zusammenhang mit entsprechenden Salzen von Verbindungen I vorstehend aufgeführt.

Die vor- und nachstehend in den Varianten beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80°C bis zur Siedetemperatur des Reaktionsmediums, vorzugsweise von etwa -10° bis etwa +200°C, und, falls erforderlich, in einem geschlossenen Gefäss, unter Druck, in einer Inertgasatmosphäre und/oder unter wasserfreien Bedingungen arbeitet. Einzelheiten zu entsprechenden Reaktionsbedingungen können insbesondere auch den Beispielen entnommen werden.

Variante a):

In die Variable $R_2$ überführbare Reste $Z_1$ sind beispielsweise Cyano, Mercapto, Halogen, die Gruppe -$N_2^+ A^-$, in der $A^-$ ein von einer Säure abgeleitetes Anion bedeutet, Amino, funktionelle Derivate von COOH, $SO_3H$, $PO_3H_2$ und $PO_2H_2$ und geschütztes 1H-Tetrazol-5-yl.

In 1H-Tetrazol-5-yl $R_2$ überführbare Reste $Z_1$ sind beispielsweise Cyano und geschütztes 1H-Tetrazol-5-yl.

Zur Herstellung von Verbindungen I, worin $R_2$ 1H-Tetrazol-5-yl bedeutet, geht man beispielsweise von Ausgangsmaterial II aus, worin $Z_1$ Cyano bedeutet, und setzt dieses, z. B. in einem inerten Lösungsmittel, wie in einem aromatischen oder araliphatischen Kohlenwasserstoff, z. B. in Toluol oder einem Xylol, vorzugsweise unter Erwärmen, mit einem Azid, z. B. mit $HN_3$ oder insbesondere einem Salz, wie Alkalimetallsalz, davon oder mit einem Organozinnazid, wie Triniederalkyl- oder Triaryl-zinnazid, um. Bevorzugte Azide sind beispielsweise Natrium- und Kaliumazid sowie Tri-$C_1$-$C_4$-alkyl-, z.B. Trimethyl- oder Tributyl-zinnazid, und Triphenylzinnazid.

Als Schutzgruppen von geschütztem 1H-Tetrazol-5-yl kommen die üblicherweise in der Tetrazolchemie verwendeten Schutzgruppen in Frage, insbesondere Triphenylmethyl, gegebenenfalls, z.B. durch Nitro, substituiertes Benzyl, wie 4-Nitrobenzyl, Niederalkoxymethyl, wie Methoxy- oder Ethoxymethyl, Niederalkylthiomethyl, wie Methylthiomethyl, sowie 2-Cyanoethyl, ferner Niederalkoxyniederalkoxymethyl, wie 2-Methoxyethoxymethyl, Benzyloxymethyl sowie Phenacyl. Die Abspaltung der Schutzgruppen erfolgt in Anlehnung an bekannte Methoden. So wird z.B. Triphenylmethyl üblicherweise durch Hydrolyse, insbesondere in Gegenwart einer Säure, z. B. in Gegenwart von Halogenwasserstoff, vorteilhaft in einem inerten Lösungsmittel, wie in einem Halogenalkan oder einem Ether, z. B. in Dichlormethan oder Dioxan, und unter Erwärmen, oder durch Hydrogenolyse in Gegenwart eines Hydrierungskatalysators, 4-Nitrobenzyl z.B. durch Hydrogenolyse in Gegenwart eines Hydrierungskatalysators, Methoxy- oder Ethoxy-methyl z.B. durch Behandeln mit einem Triniederalkyl-, wie Triethyl- oder Tributyl-zinn-bromid, Methylthiomethyl z.B. durch Behandeln mit

Trifluoressigsäure, 2-Cyanoethyl z.B. durch Hydrolyse, beispielsweise mit Natronlauge, 2-Methoxyethoxymethyl z.B. durch Hydrolyse, z.B. mit Salzsäure, und Benzyloxymethyl und Phenacyl z.B. durch Hydrogenolyse in Gegenwart eines Hydrierungskatalysators abgespalten.

Ein in $SO_3H$ $R_2$ überführbarer Rest $Z_1$ ist beispielsweise die Mercaptogruppe. Eine solche Gruppe aufweisende Ausgangsverbindungen II werden beispielsweise durch an sich bekannte Oxidationsverfahren zu Verbindungen I oxidiert, worin $R_2$ $SO_3H$ ist. Als Oxidationsmittel kommen beispielsweise anorganische Persäuren, wie Persäuren von Mineralsäuren, z.B. Periodsäure oder Perschwefelsäure, organische Persäuren, wie Percarbon- oder Persulfon-säuren, z.B. Perameisen-, Peressig-, Trifluorperessig- oder Perbenzoe-säure oder p-Toluolpersulfonsäure, oder Gemische aus Wasserstoffperoxid und Säuren, z.B. Gemische aus Wasserstoffperoxid und Essigsäure, in Betracht. Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Übergangsmetalloxide, wie Oxide von Elementen der VI. Nebengruppe, z.B. Molybdän- oder Wolfram-oxid, zu nennen sind. Die Oxidation wird unter milden Bedingungen, z.B. bei Temperaturen von etwa -50° bis etwa +100°C, durchgeführt.

Unter einer in $PO_3H_2$ $R_2$ überführbaren Gruppe ist beispielsweise eine Gruppe $-N_2^+ A^-$ zu verstehen, wobei $A^-$ für ein Anion einer Säure, wie Mineralsäure, steht. Entsprechende Diazoniumverbindungen werden beispielsweise in an sich bekannter Weise mit einem P(III)-Halogenid, wie $PCl_3$ oder $PBr_3$, umgesetzt und hydrolytisch aufgearbeitet, wobei solche Verbindungen I erhältlich sind, worin $R_2$ $PO_3H_2$ ist.

Verbindungen I, worin $R_2$ $PO_2H_2$ ist, erhält man z. B. durch in üblicher Weise erfolgende Umwandlung von $Z_1$ in einer Verbindung II, worin $Z_1$ ein funktionelles Derivat von $PO_2H_2$ ist, in $PO_2H_2$.

Als in Halogenalkansulfonylamino $R_2$ überführbarer Rest $Z_1$ kommt beispielsweise Amino in Frage. Zur Herstellung von Verbindungen I, worin $R_2$ Halogenalkansulfonylamino bedeutet, setzt man beispielsweise entsprechende Aniline mit einer üblicherweise reaktionsfähig veresterten Halogenalkansulfonsäure um, wobei gegebenenfalls in Gegenwart einer Base gearbeitet wird. Die bevorzugte reaktionsfähig veresterte Halogenalkansulfonsäure ist das entsprechende Halogenid, wie Chlorid oder Bromid.

Ein in COOH $R_2$ überführbarer Rest $Z_1$ steht beispielsweise für funktionell abgewandeltes Carboxy, wie Cyano, verestertes oder amidiertes Carboxy, Hydroxymethyl oder Formyl.

Verestertes Carboxy ist beispielsweise mit einem gegebenenfalls substituierten aliphatischen, cycloaliphatischen oder aromatischen Alkohol verestertes Carboxy. Ein aliphatischer Alkohol ist beispielsweise ein Niederalkanol, wie Methanol, Ethanol, n-Propanol, Isopropanol, n-Butanol, sek.-Butanol oder tert.-Butanol, während als cycloaliphatischer Alkohol beispielsweise ein 3- bis 8-gliedriges Cycloalkanol, wie Cyclo-pentanol, -hexanol oder -heptanol, in Frage kommt. Ein aromatischer Alkohol ist beispielsweise ein Phenol oder ein heterocyclischer Alkohol, welche jeweils substituiert sein können, insbesondere Hydroxypyridin, z.B. 2-, 3- oder 4-Hydroxypyridin.

Amidiertes Carboxy ist beispielsweise Carbamoyl, durch Hydroxy, Amino oder gegebenenfalls substituiertes Phenyl monosubstituiertes, durch Niederalkyl mono- oder disubstituiertes oder durch 4- bis 7-gliedriges Alkylen oder 3-Aza-, 3-Niederalkylaza-, 3-Oxa- oder 3-Thiaalkylen disubstituiertes Carbamoyl. Als Beispiele sind Carbamoyl, N-Mono- oder N,N-Diniederalkylcarbamoyl, wie N-Methyl-, N-Ethyl-, N,N-Dimethyl-, N,N-Diethyl- und N,N-Dipropyl-carbamoyl, Pyrrolidino- und Piperidinocarbonyl, Morpholino-, Piperazino-, 4-Methylpiperazino- und Thiomorpholino-carbonyl, Anilinocarbonyl und durch Niederalkyl, Niederalkoxy und/oder Halogen substituiertes Anilinocarbonyl zu nennen.

Bevorzugtes funktionell abgewandeltes Carboxy ist Niederalkoxycarbonyl, wie Methoxy-oder Ethoxycarbonyl, und Cyano.

Verbindungen I, worin $R_2$ Carboxy ist, können beispielsweise ausgehend von Verbindungen II, worin $Z_1$ Cyano oder verestertes oder amidiertes Carboxy bedeutet, durch Hydrolyse, insbesondere in Gegenwart einer Base, oder, ausgehend von Verbindungen II, worin $Z_1$ Hydroxymethyl oder Formyl bedeutet, durch Oxidation hergestellt werden. Die Oxidation erfolgt beispielsweise in einem inerten Lösungsmittel, wie in einer Niederalkancarbonsäure, z.B. Essigsäure, einem Keton, z.B. Aceton, einem Ether, z.B. Tetrahydrofuran, einem heterocyclischen Aromaten, z.B. Pyridin, oder in Wasser, oder in einem Gemisch davon, erforderlichenfalls unter Kühlen oder Erwärmen, z.B. in einem Temperaturbereich von etwa 0° bis etwa +150°C. Als Oxidationsmittel kommen beispielsweise oxidierende Übergangsmetallverbindungen, insbesondere solche mit Elementen der I., VI. oder VII. Nebengruppe, in Frage. Als Beispiele seien genannt: Silberverbindungen, wie Silber-nitrat, -oxid und -picolinat, Chromverbindungen, wie Chromtrioxid und Kaliumdichromat, und Manganverbindungen, wie Kalium-, Tetra-butylammonium- und Benzyltriethylammonium-permanganat. Weitere Oxidahonsmittel sind beispielsweise geeignete Verbindungen mit Elementen der IV. Hauptgruppe, wie Bleidioxid, oder Halogen-Sauerstoff-Verbindungen, wie Natriumiodat oder Kaliumperiodat.

Das Ausgangsmaterial II ist in Analogie zu bekannten Methoden zugänglich, beispielsweise indem man eine Säure der Formel $H_2N$-X-COOH (IIa) oder einen Ester der Formel $H_2N$-X-COOE (IIa'; -COOE = Ester-

gruppe, z. B. Niederalkoxycarbonyl, wie Methoxy- oder Ethoxy-carbonyl) in Gegenwart einer Base mit einer Verbindung der Formel $R_1$-C(=O)-$Z_5$ (IIb; $Z_5$ = nucleofuge Abgangsgruppe, z. B. Halogen, wie Chlor) umsetzt, gegebenenfalls im Reaktionsprodukt die Estergruppe -COOE verseift, die resultierende Säure der Formel $R_1$ -C(=O)-NH-X-COOH (IIc), gegebenenfalls nach Ueberführung in ein reaktives Säurederivat, z. B. nach Umsetzung mit N-Hydroxysuccinimid, in Gegenwart eines wasserbindenden Mittels, wie eines Carbodiimids, z. B. in Gegenwart von N,N'-Dicyclohexylcarbodiimid, vorteilhaft in einem inerten Lösungsmittel, z. B. in einem N,N-Diniederalkylniederalkansäureamid, wie in N,N-Dimethylformamid, mit einer Verbindung der Formel

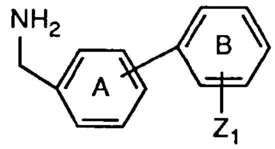

(IId)

zu einer Verbindung der Formel

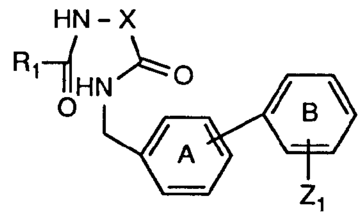

(IIe)

umsetzt, die schliesslich, z. B. in Gegenwart einer Säure, wie einer organischen Sulfonsäure, z. B. in Gegenwart von p-Toluolsulfonsäure, und gegebenenfalls unter Erwärmen, zu einer Verbindung II cyclisiert wird.

Gemäss einer bevorzugten Ausführungsform der Variante a) kann man ohne Isolierung der Verbindungen II von Verbindungen IIe, worin $Z_1$ Cyano ist, direkt zu Verbindungen I, worin $R_2$ 1H-Tetrazol-5-yl ist, gelangen, indem man entsprechende Verbindungen IIe, z. B. in einem inerten Lösungsmittel, wie in einem aromatischen oder araliphatischen Kohlenwasserstoff, z. B. in Toluol oder einem Xylol, vorzugsweise unter Erwärmen, mit einem Azid, z. B. mit $HN_3$ oder insbesondere einem Salz, wie Alkalimetallsalz, davon oder mit einem Organozinnazid, wie Triniederalkyl- oder Triaryl-zinnazid, umsetzt. Bevorzugt sind dabei die vorstehend angegebenen Azide.

Das Ausgangsmaterial II ist auch erhältlich, indem man eine Verbindung V mit einer Verbindung IId umsetzt, z. B. in einem inerten Lösungsmittel, wie in einem aromatischen oder araliphatischen Kohlenwasserstoff, z. B. in Toluol oder einem Xylol, und vorzugsweise unter Erwärmen, und die resultierende Verbindung der Formel

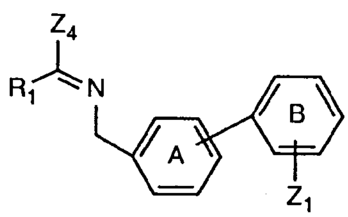

(IIf)

mit einer Verbindung IIa' umsetzt, z. B. in einem inerten Lösungsmittel, wie in einem aromatischen oder araliphatischen Kohlenwasserstoff, z. B. in Toluol oder einem Xylol, und vorzugsweise unter Erwärmen sowie gegebenenfalls in Gegenwart eines basischen Mittels, wie in Gegenwart von Pyridin oder Triethylamin.

Weiterhin ist das Ausgangsmaterial II auch dadurch erhältlich, dass man eine Verbindung V mit einer Verbindung IIa' reagieren lässt, z. B. in einem inerten Lösungsmittel, wie in Toluol oder einem Xylol, und gegebenenfalls in Gegenwart eines basischen Mittels, wie in Gegenwart von pyridin oder Triethylamin, die resultierende Verbindung der Formel

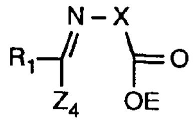

(IIn)

mit einer Verbindung IId, z. B. in einem inerten Lösungsmittel, wie in Toluol oder einem Xylol, und gegebenenfalls in Gegenwart eines basischen Mittels, wie in Gegenwart von Pyridin oder Triethylamin, zu einer Verbindung der Formel IIm

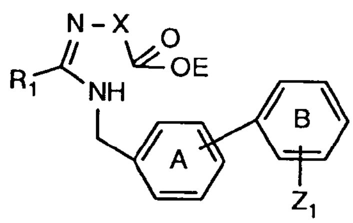

(IIm)

umsetzt, welche dann zu einer Verbindung II cyclisiert wird, z. B. in einem inerten Lösungsmittel, wie in einem aromatischen oder araliphatischen Kohlenwasserstoff, z. B in Toluol oder einem Xylol, und vorzugsweise unter Erwärmen.

Schliesslich kann man das Ausgangsmaterial II auch dadurch erhalten, dass man eine Verbindung der Formel

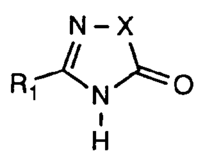

(IIh)

mit einer Verbindung der Formel

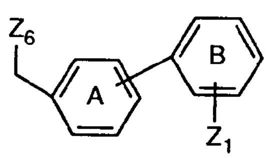

(IIi),

worin $Z_6$ für eine nucleofuge Abgangsgruppe, z. B. Halogen, wie Chlor oder Brom, steht, umsetzt, z. B. in einem inerten Lösungsmittel, wie in einem N,N-Diniederalkylniederalkansäureamid, z. B. in N,N-Dimethylformamid, und vorzugsweise in Gegenwart eines basischen Mittels, wie eines Alkalimetallhydrids, z. B. in Gegenwart von Natrium- oder Kalium-hydrid.

Eine Verbindung IIh erhält man z. B. durch Umsetzung einer Verbindung der Formel $H_2N$-X-C(=O)-$NH_2$ (IIg) mit einer Verbindung V, z. B. in einem inerten Lösungsmittel, wie in Toluol oder einem Xylol, und vorzugsweise unter Erwärmen sowie gegebenenfalls in Gegenwart eines basischen Mittels, wie in Gegenwart von Pyridin oder Triethylamin, oder durch Umsetzung einer Verbindung der Formel $R_1$-C(=NH)-Zr (IIj; $Z_7$= nucleofuge Abgangsgruppe, vorzugsweise Niederalkoxy, wie Methoxy oder Ethoxy) mit einer Verbindung IIa′, z. B. in einem inerten Lösungsmittel, wie in einem aromatischen oder araliphatischen Kohlenwasserstoff, z. B. in Toluol oder einem Xylol, und vorzugsweise unter Erwärmen sowie gegebenenfalls in Gegenwart eines basischen Mittels, wie in Gegenwart von pyridin oder Triethylamin, oder durch Umsetzung einer Verbindung der Formel $R_1$-C(=NH)-$NH_2$ (IIk) mit einer Verbindung IIa′ oder, zur Herstellung von Verbindungen IIh, worin X eine Gruppe der Formel -C($R_3$)$R_4$-CH($R_5$)- (Ia′) ist, mit einer Verbindung der Formel ($R_3$)$R_4$C=C($R_5$)-C(=O)-OE (IIo; -COOE = Estergruppe, z. B. Niederalkoxycarbonyl, wie Methoxy- oder Ethoxy-carbonyl), gegebenenfalls in einem inerten Lösungsmittel, wie in Toluol oder einem Xylol, sowie gegebenenfalls unter Erwärmen.

Variante b):

Die Cyclisierung erfolgt in an sich bekannter Weise, beispielsweise in einem inerten Lösungsmittel, wie in einem aromatischen oder araliphatischen Kohlenwasserstoff, z. B. in Toluol oder einem Xylol, vorzugsweise unter Erwärmen, sowie gegebenenfalls in Gegenwart eines basischen Mittels, wie in Gegenwart von Pyridin oder Triethylamin, oder eines sauren Mittels, z. B. in Gegenwart einer Säure, wie einer Mineralsäure oder einer organischen Carbon- oder Sulfon-säure, z. B. in Gegenwart von einer Halogenwasserstoffsäure, wie Chlorwasserstoffsäure, oder von Essigsäure oder p-Toluolsulfonsäure.

Das Ausgangsmaterial III kann in an sich bekannter Weise hergestellt werden, beispielsweise durch Umsetzung einer Säure der Formel $R_1$-C(=O)-NH-X-COOH (IIc), gegebenenfalls nach Ueberführung in ein reaktives Säurederivat, z. B. nach Umsetzung mit N-Hydroxysuccinimid, in Gegenwart eines wasserbindenden Mittels, wie eines Carbodiimids, z. B. in Gegenwart von N,N'-Dicyclohexylcarbodiimid, vorteilhaft in einem inerten Lösungsmittel, z. B. in einem N,N-Diniederalkylniederalkansäureamid, wie in N,N-Dimethylformamid, mit einer Verbindung der Formel

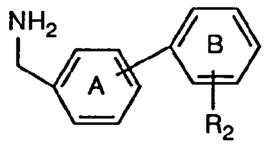

(IIIa),

wobei Verbindungen IIc z. B. wie in der Variante a) beschrieben erhalten werden können.

Variante c) :

Gegebenenfalls funktionell abgewandeltes Oxo ($Z_2$ und $Z_3$ gemeinsam) ist z. B. Oxo, Thioxo oder gegebenenfalls substituiertes Imino, wie Imino, N-Niederalkylimino, N-Benzoylimino oder N-Niederalkansulfonylimino.

Eine nucleofuge Abgangsgruppe $Z_2$ bzw. $Z_3$ bzw. $Z_4$ ist z. B. Hydroxy, Niederalkoxy, wie Methoxy oder Ethoxy, Niederalkylthio, wie Methylthio, gegebenenfalls substituiertes Amino, wie Amino, N-Niederalkylamino oder N,N-Diniederalkylamino, oder Sulfonyloxy, wie gegebenenfalls halogeniertes Niederalkansulfonyloxy oder gegebenenfalls substituiertes Benzolsulfonyloxy, wie Methan-, Ethan-, Trifluormethan-, Benzol- oder p-Toluol-sulfonyloxy. Bevorzugt sind Verbindungen V, worin $Z_2$, $Z_3$ und $Z_4$ Niederalkoxy, wie Methoxy oder Ethoxy, bedeuten, d. h. $R_1$-substituierte Orthoester.

Die Umsetzung einer Verbindung IV mit einer Verbindung V erfolgt in Analogie zu bekannten Methoden unter den üblichen Reaktionsbedingungen, z. B. in einem inerten Lösungsmittel, wie in einem aromatischen oder araliphatischen Kohlenwasserstoff, z. B. in Toluol oder einem Xylol, bei Raumtemperatur oder unter Erwärmen und gegebenenfalls in Gegenwart eines sauren Mittels, z. B. in Gegenwart einer Säure, wie einer Mineralsäure oder einer organischen Carbon- oder Sulfon-säure, z. B. in Gegenwart von einer Halogen-wasserstoffsäure, wie Chlorwasserstoffsäure, oder von Essigsäure oder p-Toluolsulfonsäure.

Das Ausgangsmaterial IV kann in an sich bekannter Weise erhalten werden, z. B. durch Umsetzung einer Säure der Formel $H_2N$-X-C(=O)-OH (IIa), gegebenenfalls nach Ueberführung in ein reaktives Säurederivat, z. B. nach Umsetzung mit N-Hydroxysuccinimid, in Gegenwart eines wasserbindenden Mittels, wie eines Carbodiimids, z. B. in Gegenwart von N,N'-Dicyclohexylcarbodiimid, vorteilhaft in einem inerten Lösungsmittel, z. B. in einem N,N-Diniederalkylniederalkansäureamid, wie in N,N-Dimethylformamid, mit einer Verbindung der Formel

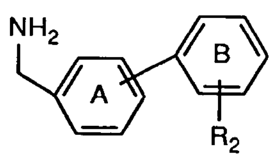

(IIIa).

Das Ausgangsmaterial V ist bekannt oder kann in Analogie zu bekannten Methoden hergestellt werden.

Eine verfährensgemäss oder auf andere Weise erhältliche Verbindung I oder ein Tautomeres davon kann in an sich bekannter Weise in eine andere Verbindung I oder ein Tautomeres davon überführt werden.

Beispielsweise kann eine Hydroxy aufweisende Verbindung I nach an sich bekannten Methoden verethert werden. Die Veretherung kann z.B. mit einem Alkohol, wie einem Niederalkanol, oder einem reaktionsfähigen

Ester desselben erfolgen. Als reaktionsfähige Ester der gewünschten Alkohole kommen beispielsweise solche mit starken anorganischen oder organischen Säuren in Frage, wie entsprechende Halogenide, Sulfate, Niederalkansulfonate oder gegebenenfalls substituierte Benzolsulfonate, z.B. Chloride, Bromide, Iodide oder Methan-, Benzol- oder p-Toluol-sulfonate. Die Veretherung kann z.B. in Gegenwart einer Base, z. B. eines Alkalimetall-hydrids, -hydroxids oder -carbonats oder eines basischen Amins, erfolgen. Umgekekehrt können entsprechende Ether, wie Niederalkoxyverbindungen, z.B. mittels starker Säuren, wie Mineralsäuren, z.B. Brom-oder Iod-wasserstoffsäure, die vorteilhaft in Form von Pyridiniumhalogeniden vorliegen können, oder mittels Lewissäuren, z.B. Halogeniden von Elementen der III. Hauptgruppe oder der entsprechenden Nebengruppen, gespalten werden. Diese Umsetzungen können, falls erforderlich, unter Kühlen oder Erwärmen, z.B. in einem Temperaturbereich von etwa -20° bis etwa +100°C, in An- oder Abwesenheit eines Lösungs- oder Verdünnungsmittels, unter Inertgas und/oder unter Druck und gegebenenfalls in einem geschlossenen Gefäss durchgeführt werden.

Weist eine der Variablen Amino auf, können entsprechende Verbindungen I in an sich bekannter Weise N-(ar)alkyliert werden; ebenso können Carbamoyl bzw. Carbamoyl aufweisende Reste N-(ar)alkyliert werden. Die (Ar-)Alkylierung erfolgt z.B. mit einem (Aryl-)$C_1$-$C_7$-Alkyl-halogenid, z.B. -bromid oder -iodid, (Aryl-)$C_1$-$C_7$-Alkansulfonat, z.B mit Methansulfonat oder p-Toluolsulfonat, oder einem Di-$C_1$-$C_7$-alkylsulfat, z.B. Dimethylsulfat, vorzugsweise unter basischen Bedingungen, wie in Gegenwart von Natronlauge oder Kalilauge, und vorteilhaft in Gegenwart eines Phasentransfer-Katalysators, wie von Tetrabutylammoniumbromid oder Benzyltrimethylammonium-chlorid, wobei indes stärker basische Kondensationsmittel, wie Alkalimetall-amide, -hydride oder -alkoholate, z.B. Namtumamid, Natriumhydrid oder Natriumethanolat, erforderlich sein können.

In Verbindungen I, die als Substituenten eine veresterte oder amidierte Carboxygruppe aufweisen, kann man eine solche Gruppe, z.B. mittels Hydrolyse, z.B. in Gegenwart eines basischen Mittels oder eines sauren Mittels, wie einer Mineralsäure, in eine freie Carboxygruppe überführen.

Ferner kann man in Verbindungen I, die als Substituenten eine Carboxygruppe aufweisen (insbesondere, sofern $R_2$ von Carboxy verschieden ist), diese, z.B. durch Behandeln mit einem Alkohol, wie einem Niederalkanol, in Gegenwart eines geeigneten Veresterungsmittels, wie eines sauren Reagens, z.B. einer anorganischen oder organischen Säure oder einer Lewissäure, z.B. von Zinkchlorid, oder eines wasserbindenden Kondensationsmittels, z.B. eines Carbodiimids, wie von N,N'-Dicyclohexylcarbodiimid, oder durch Behandeln mit einem Diazoreagens, wie mit einem Diazoniederalkan, z.B. Diazomethan, in eine veresterte Carboxygruppe überführen. Diese kann man auch erhalten, wenn man Verbindungen I, worin die Carboxygruppe in freier Form oder in Salz-, wie Ammonium-oder Metall-, z.B. Alkalimetall-, wie Natrium- oder Kalium-salzform vorliegt, mit einem $C_1$-$C_7$-Alkylhalogenid, z.B. Methyl- oder Ethyl-bromid oder -iodid, oder einem organischen Sulfonsäureester, wie einem entsprechenden $C_1$-$C_7$-Alkylester, z.B. Methansulfonsäure- oder p-Toluolsulfonsäure-methylester oder -ethylester, behandelt.

Verbindungen I, die als Substituenten eine veresterte Carboxygruppe aufweisen, kann man durch Umesterung, z.B. durch Behandeln mit einem Alkohol, üblicherwiese mit einem höheren als dem der veresterten Carboxygruppe im Ausgangsmaterial entsprechenden Alkohol, in Gegenwart eines geeigneten Umesterungsmittels, wie eines basischen Mittels, z.B. eines Alkalimetall-$C_1$-$C_7$-alkanoats, -$C_1$-$C_7$-alkanolats oder -cyanids, wie von Natrium-acetat, -methanolat, -ethanolat, -tert.-butanolat oder -cyanid, oder eines geeigneten sauren Mittels, gegebenenfalls unter Entfernung des entstehenden Alkohols, z.B. durch Destillation, in andere Esterverbindungen I umwandeln. Man kann auch von entsprechenden, sogenannten aktivierten Estern I ausgehen, die als Substituenten eine aktivierte veresterte Carboxygruppe aufweisen (siehe unten), und diese durch Behandeln mit einem $C_1$-$C_7$-Alkanol in einen anderen Ester umwandeln.

Man kann in Verbindungen I, die als Substituenten die Carboxygruppe enthalten, diese auch zuerst in ein reaktionsfähiges Derivat, wie ein Anhydrid (auch ein gemischtes Anhydrid), ein Säure-halogenid, z.B. -chlorid (z.B. durch Behandeln mit einem Thionyl-halogenid, z.B. -chlorid), ein Anhydrid mit einem Ameisensäure-ester, z.B. -$C_1$-$C_7$-alkylester (z.B. durch Behandeln eines Salzes, wie eines Ammonium- oder Alkalimetallsalzes, mit einem Halogen-, wie Chlor-ameisensäureester, wie $C_1$-$C_7$-Alkylester), oder einen aktivierten Ester, wie Cyanmethyl-, Nitrophenyl-, z.B. 4-Nitro-phenyl-, oder Polyhalogenphenyl-, z.B. Pentachlorphenyl-ester (z.B. durch Behandeln mit einer entsprechenden Hydroxyverbindung in Gegenwart eines geeigneten Kondensationsmittels, wie von N,N'-Dicyclohexylcarbodiimid) überführen, und ein solches reaktionsfähiges Derivat dann mit einem Amin umsetzen und so zu Amidverbindungen I gelangen, die als Substituenten eine amidierte Carboxygruppe aufweisen. Dabei kann man diese direkt oder über Zwischenverbindungen erhalten; so kann man z.B. einen aktivierten Ester, wie einen 4-Nitrophenylester, einer Verbindung I mit einer Carboxygruppe zuerst mit einem 1-unsubstituierten Imidazol umsetzen und die so entstandene 1-Imidazolylcarbonyl-verbindung mit einem Amin in Reaktion bringen. Man kann aber auch andere, nicht-aktivierte Ester, wie $C_1$-$C_7$-Alkylester, von Verbindungen 1 mit Aminen zur Reaktion bringen.

Weist ein aromatischer Ring als Substituenten ein Wasserstoffatom auf, so kann dieses mit Hilfe eines

Halogenierungsmittels in üblicher Weise durch ein Halogenatom ersetzt werden, z.B. mit Brom, Hypobromsäure, einem Acylhypobromit oder einer anderen organischen Bromverbindung, z.B. N-Bromsuccinimid, N-Bromacetamid, N-Bromphthalimid, Pyridiniumperbromid, Dioxandibromid, 1,3-Dibrom-5,5-dimethylhydantoin oder 2,4,4,6-Tetrabrom-2,5-cyclohexandien-1-on, durch Brom oder mit elementarem Chlor, z.B. in einem halogenierten Kohlenwasserstoff, wie Chloroform, und unter Kühlen, z.B. bis auf etwa - 10°C, durch Chlor.

Enthält ein aromatischer Ring eine Aminogruppe, so kann diese in üblicher Weise diazotiert werden, z.B. durch Behandeln mit einem Nitrit, z.B. Natriumnitrit, in Gegenwart einer geeigneten Protonsäure, z.B. einer Mineralsäure, wobei die Reaktions-temperatur vorteilhaft unter etwa 5°C gehalten wird. Die so erhältliche, in Salzform vorliegende Diazoniumgruppe kann man nach üblichen Verfahren beispielsweise wie folgt substituieren: durch die Hydroxygruppe analog der Phenolverkochung in Gegenwart von Wasser, durch eine Alkoxygruppe durch Behandeln mit einem entsprechenden Alkohol, wobei Energie zugeführt werden muss; durch das Fluoratom analog der Schiemann-Reaktion bei der Thermolyse von entsprechenden Diazoniumtetrafluorboraten; oder durch Chlor, Brom, Iod oder die Cyanogruppe analog der Sandmeyer-Reaktion durch Umsetzung mit entsprechenden Cu(I)-Salzen, zunächst unter Kühlen, z.B. auf unter etwa 5°C, und anschliessendem Erhitzen, z.B. auf etwa 60° bis etwa 150°C.

Enthalten die Verbindungen I ungesättigte Reste, wie Niederalkenyl- oder Niederalkinyl-gruppierungen, können diese in an sich bekannter Weise in gesättigte Reste überführt werden. So erfolgt beispielsweise die Hydrierung von Mehrfachbindungen durch katalytische Hyrierung in Gegenwart von Hydrierungskatalysatoren, wobei hierfür z.B. Nickel, wie Raney-Nickel, sowie Edelmetalle oder deren Derivate, z.B. Oxide, geeignet sind, wie Palladium oder Platinoxid, die gegebenenfalls auf Trägermaterialien, z.B. auf Kohle oder Calciumcarbonat, aufgezogen sein können. Die Hydrierung kann vorzugsweise bei Drucken zwischen etwa 1 und etwa 100 at und bei Temperaturen zwischen etwa -80° und etwa +200°C, vor allem zwischen Raumtemperatur und etwa 100°C, durchgeführt werden. Die Reaktion erfolgt zweckmässig in einem Lösungsmittel, wie Wasser, einem Niederalkanol, z.B. Ethanol, Isopropanol oder n-Butanol, einem Ether, z.B. Dioxan, oder einer Niederalkancarbonsäure, z.B. Essigsäure.

Die Erfindung betrifft insbesondere die in den Beispielen beschriebenen Verfahren.

Salze von Verbindungen I können in an sich bekannter Weise hergestellt werden. So erhält man beispielsweise Säureadditionssalze von Verbindungen I durch Behandeln mit einer geeigneten Säure oder einem geeigneten Ionenaustauscherreagens. Salze von Verbindungen I können in üblicher Weise in die freien Verbindungen I überführt werden, Säureadditionssalze z.B. durch Behandeln mit einem geeigneten basischen Mittel oder einem geeigneten Ionenaustauscherreagens.

Salze von Verbindungen I können in an sich bekannter Weise in andere Salze von Verbindungen I umgewandelt werden.

Je nach Verfahrensweise bzw. Reaktionsbedingungen können die Verbindungen I mit salzbildenden, insbesondere basischen Eigenschaften, in freier Form oder in Form von Salzen erhalten werden.

Infolge der engen Beziehung zwischen der Verbindung I in freier Form und in Form ihrer Salze sind im Vorausgegangenen und nachfolgend unter der freien Verbindung I bzw. ihren Salzen sinn- und zweckgemäss gegebenenfalls auch die entsprechenden Salze bzw. die freie Verbindung I zu verstehen.

Die Verbindungen I einschliesslich ihrer Salze von salzbildenden Verbindungen können auch in Form ihrer Hydrate erhalten werden und/oder andere, z. B. zur Kristallisation verwendete, Lösungsmittel einschliessen.

Die Verbindungen I und ihre Salze können, je nach der Wahl der Ausgangsstoffe und Arbeitsweisen, in Form eines der möglichen Isomeren oder als Gemisch derselben, z.B. je nach Anzahl, absoluter und relativer Konfiguration der asymmetrischen Kohlenstoffatome als reine Isomere, wie Antipoden und/oder Diastereomere, oder als Isomeren gemische, wie Enantiomerengemische, z. B. Racemate, Diastereomerengemische oder Racematgemische, vorliegen.

Erhaltene Diastereomerengemische und Racematgemische können auf Grund der physikalisch-chemischen Unterschiede der Bestandteile in bekannter Weise in die reinen Diastereomeren oder Racemate aufgetrennt werden, beispielsweise durch fraktionierte Kristallisation. Erhaltene Enantiomerengemische, wie Racemate, lassen sich nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Überführung in diastereomere Salze, z.B. durch Umsetzung eines basischen Endstoffracemats mit einer optisch aktiven Säure, wie Carbonsäure, z.B. Wein-oder Äpfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausfürhungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsgedingungen bildet.

Beim Verfahren der vorliegenden Erfindung werden vorzugsweise solche Ausgangsstoffe und Zwischenprodukte verwendet, welche zu den eingangs als besonders wertvoll geschilderten Verbindungen I führen. Neue Ausgangsstoffe und Zwischenprodukte für die Herstellung der Verbindungen I, ihre Verwendung und ein Verfahren zu ihrer Herstellung bilden ebenfalls einen Gegenstand der Erfindung, wobei die Variablen $R_1$, $R_2$ und X und die Ringe A und B die für die Verbindungen I angegebenen Bedeutungen haben.

Die Verbindungen I und ihre pharmazeutisch verwendbaren Salze können, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Antihypertensiva, verwendet werden.

Die Erfindung betrifft daher gleichfalls pharmazeutische Präparate, die eine Verbindung I in freier Form oder in Form eines pharmazeutisch verwendbaren Salzes als Wirkstoff enthalten, sowie ein Verfahren zu ihrer Herstellung. Bei diesen pharmazeutischen Präparaten handelt es sich um solche zur enteralen, wie oralen, ferner rektalen oder parenteralen Verabreichung an Warmblüter, wobei der pharmakologische Wirkstoff allein oder zusammen mit üblichen pharmazeutischen Hilfsstoffen enthalten ist. Die pharmazeutischen Präparate enthalten z.B. von etwa 0,1 % bis 100 %, vorzugsweise von etwa 1 % bis etwa 60 %, des Wirkstoffs. Pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren, hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragantgummi, Methylcellulose und/oder Polyvinylpyrrolidon, und, wenn erwünscht, Sprengmittel, wie die obengenannten Stärken, ferner Carboxymethylstärke, quervemetztes Polyvinylpyrrolidon, Agar oder Alginsäure oder ein Salz davon, wie Natriumalginat. Hilfsmittel sind in erster Linie Fliessregulier- und Schmierminel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaftresistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeurische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole und höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole und Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet, oder wässrige Injektionssuspensionen, welche viskositätserhöhende Stoffe, z.B. Natriumcarboxymethylcellulose, Sorbit und/oder

Dextran, und gegebenenfalls auch Stabilisatoren enthalten.

Die Dosierung des Wirkstoffes kann von verschiedenen Faktoren, wie Applikationsweise, Warmblüter-Spezies, Alter und/oder individuellem Zustand, abhängen. Im Normalfall ist für einen etwa 75 kg schweren Patienten bei oraler Applikation eine ungefähre Tagesdosis von etwa 10 mg bis etwa 250 mg zu veranschlagen.

Die nachfolgenden Beispiele illustrieren die oben beschriebene Erfindung; sie sollen jedoch diese in ihrem Umfang in keiner Weise einschränken. Temperaturen sind in Grad Celsius angegeben.

Beispiel 1: Eine Lösung von 7.55 g (20 mmol) 2-Methyl-2-pentanoylamino-propansäure-N-(2′-cyanobiphenyl-4-ylmethyl)amid und 10.6 g (32 mmol) Tributylzinnazid in 100 ml o-Xylol wird unter Rühren 40 Stunden zum Rückfluss erhitzt. Nach dem Abkühlen wird das Reaktionsgemisch mit 80 ml 1 N-Natronlauge versetzt und 2 bis 3 Stunden gerührt. Die wässrige Phase wird abgetrennt und mit 1 N-Salzsäure auf pH 3 bis 4 gebracht. Das sich abscheidende Oel wird mit Ethylacetat extrahiert und das so erhaltene Rohprodukt durch Flash-Chromatographie gereinigt (Kieselgel 60, 40-63 µm, Toluol/Isopropanol/Eisessig = 170/30/2). Die in diesem System einen $R_f$-Wert von 0.29 aufweisenden Fraktionen werden vereinigt und eingedampft und der Rückstand aus Isopropanol umkristallisiert. Man erhält so 2-(n-Butyl)-4,4-dimethyl-5-oxo-1-[2′-(1H-tetrazol-5-yl)-biphenyl-4-ylmethyl]-4,5-dihydro- 1H-imidazol vom Smp. 181-182°.

Das Ausgangsmaterial kann beispielsweise wie folgt hergestellt werden:

a) 2-Amino-2-methyl-propansäuremethylester-hydrochlorid und Pentanoylchlorid werden in Dioxan/Hünigbase zu 2-Methyl-2-pentanoylamino-propansäuremethylester umgesetzt. Dieser wird roh in Dioxan mit 2 N:Natronlauge zu 2-Methyl-2-pentanoylamino-propansäure [Smp.: 144-148° (aus Ethylacetat)] hydrolysiert.

b) Eine Lösung von 7.5 g (40 mmol) 2-Methyl-2-pentanoylamino-propansäure in 100 ml N,N-Dimethylformamid wird mit 7.5 g (63 mmol) N-Hydroxysuccinimid und 9.6 g (47 mmol) N,N′-Dicyclohexylcarbodiimid versetzt. Das Gemisch wird 15 Minuten gerührt, dann mit einer Lösung von 8.3 g (40mmol) 4-Aminomethyl-2′-cyano-biphenyl in 20 ml N,N-Dimethylformamid versetzt und anschliessend 24 Stunden bei Raumtemperatur gerührt. Der entstandene Niederschlag wird abfiltriert und das Filtrat im Vakuum eingedampft. Der Eindampfrückstand wird zwischen Ethylacetat und 2 N-Natronlauge verteilt und die organische Phase abgetrennt, getrocknet und eingedampft. Man erhält so 2-Methyl-2-pentanoylamino-propansäure-N-(2′-cyanobiphenyl-4-ylmethyl)amid als gelbes Oel, das allmählich kristallin erstarrt und roh weiterverwendet werden kann.

Beispiel 2: Eine Lösung von 1.3 g (2 mmol) 2-(n-Butyl)-4,4-dimethyl-5-oxo-1-[2′-(1-triphenylmethyl- 1H-tetrazol-5-yl)biphenyl-4-ylmethyl]-4,5-dihydro-1H-imidazol in 30 ml Dioxan wird mit 5 ml 2 N-Salzsäure versetzt und das Gemisch 2 bis 3 Stunden auf 80° erwärmt. Das Dioxan wird abgedampft und der Rückstand zwischen 10 ml 2 N-Natronlauge und 30 ml Ethylacetat verteilt. Aufarbeitung des Natronlauge-Extrakts analog Beispiel 1 ergibt 2-(n-Butyl)-4,4-dimethyl-5-oxo-1-[2′-(1H-tetrazol-5-yl)biphenyl-4-ylmethyl]-4,5-dihydro-1H-imidazol [Smp.: 181-182° (aus Isopropanol)].

Das Ausgangsmaterial kann beispielsweise wie folgt erhalten werden:

a) 1-Ethoxy-1-imino-pentan und 2-Amino-2-methyl-propansäureethylester werden zu 2-(n-Butyl)-4,4-dimethyl-5-oxo-4,5-dihydro-1H-imidazol umgesetzt, das als Oel erhalten wird [$R_f$ = 0.33 (Toluol/Isopropanol/konzentriertes Ammoniak = 170/30/2)].

b) Eine Lösung von 3.4 g (20 mmol) 2-(n-Butyl)-4,4-dimethyl-5-oxo-4,5-dihydro-1H-imidazol in 50 ml N,N-Dimethylformamid wird portionsweise unter Rühren bei 5 bis 10° mit 0.95 g (20 mmol) Natriumhydrid-Suspension (50 % in Oel) versetzt. Das Gemisch wird 1 Stunde bei Raumtemperatur nachgerührt und dann mit einer Lösung von 5.6 g (10 mmol) 2′-(1-Triphenylmethyl-1H-tetrazol-5-yl)biphenyl-4-ylmethylbromid in 20 ml N,N-Dimethylformamid versetzt. Das Reaktionsgemisch wird 18 bis 24 Stunden bei 20° Innentemperatur gerührt, dann mit Essigsäure neutralisiert und nach Abdampfen des Lösungsmittels zwischen Ethylacetat und Wasser verteilt. Durch Eindampfen der organischen Phase erhält man 2-(n-Butyl)-4,4-dimethyl-5-oxo-1-[2′-(1-triphenyl-methyl- 1H-tetrazol-5-yl)biphenyl-4-ylmethyl]-4,5-dihydro-1H-imidazol, welches roh weiterverwendet wird.

Beispiel 3: Eine Lösung von 2.4 g (6 mmol) 2-(n-Butyl)-3-(2′-cyanobiphenyl-4-ylmethyl)-4-oxo-1,3-diazaspiro[4.5]dek-1-en und 3.6 g (10.8 mmol) Tributylzinnazid in 50 ml o-Xylol wird 36 Stunden unter Rückfluss erhitzt. Aufarbeitung analog Beispiel 1 unter Verwendung von 50 ml 1 N-Kalilauge ergibt ein Oel, aus dem nach Zugabe von wenig Ethylacetat 2-(n-Butyl)-4-oxo-3-[2′-(1H-tetrazol-5-yl)biphenyl-4-ylmethyl]-1,3-diazaspiro[4.5]dek-1-en [Smp.: 145-153° (Sintern ab 112°)] auskristallisiert.

Das Ausgangsmaterial kann beispielsweise wie folgt hergestellt werden:

a) Eine Suspension von 14.3 g (100 mmol) 1-Amino-1-carboxy-cyclohexan und 26 g (200 mmol) Hünigbase in 400 ml Tetrahydrofuran wird bei Raumtemperatur innert 15 Minuten unter Rühren mit 14.5 g (120 mmol) Pentanoylchlorid versetzt. Das Gemisch wird 20 bis 25 Stunden gerührt, das Lösungsmittel abgedampft, der Rückstand mit 100 ml Wasser versetzt und das Gemisch 1 Stunde vemührt. Der kristalline Rückstand

wird abfiltriert und im Vakuum getrocknet. Das so erhaltene rohe 1-Carboxy- 1 -pentanoylamino-cyclohexan schmilzt bei 148- 154° und kann ohne weitere Reinigung weiterverarbeitet werden.

b) Ein Gemisch aus 4.55 g (20 mmol) 1-Carboxy-1-pentanoylamino-cyclohexan, 3.45 g (30 mmol) N-Hydroxysuccinimid, 4.74 g (23 mmol) N,N′-Dicyclohexylcarbodiimid und 50 ml N,N-Dimethylformamid wird 20 Minuten bei Raumtemperatur gerührt. Zu der entstandenen Suspension gibt man eine Lösung von 4.2 g (20 mmol) 4-Aminomethyl-2′-cyano-biphenyl in 10 ml N,N-Dimethylformamid und rührt das Gemisch 24 Stunden. Aufarbeitung analog Beispiel 1 b) führt zu 1 -(2′-Cyanobiphenyl-4-ylmethylamino-carbonyl)-1-pentanoylamino-cyclohexan, das allmählich kristallin erstarrt und roh weiterverarbeitet werden kann. Eine aus Ethylacetat umkristallisierte Probe schmilzt bei 135-137°.

c) Ein Gemisch aus 5.9 g (14 mmol) rohem 1-(2′-Cyanobiphenyl-4-ylmethylaminocarbonyl)-1-pentanoylamino-cyclohexan, 2.8 g (14 mmol) p-Toluolsulfonsäwemonohydrat und 150 ml Xylol-Gemisch wird am Wasserabscheider unter Rückfluss erhitzt. Nach 3 Stunden wird der Wasserabscheider durch einen absteigenden Kühler ersetzt und 50 ml Xylol werden abdestilliert. Das restliche Lösungsmittel wird im Vakuum (ca. 1 mm Hg) abdestilliert. Der Rückstand wird zwischen 20 ml 2 N-Natronlauge und 200 ml Ethylacetat verteilt. Der nach Abdampfen des Ethylacetats erhaltene Rückstand wird an Silicagel mit Hexan/Ethylacetat (7/2) flash-chromatographiert. Die das Produkt enthaltenden Fraktionen ($R_f$ = 0. 12) werden vereinigt und eingedampft und liefem so 2-(n-Butyl)-3-(2′-cyanobiphenyl-4-ylmethyl)-4-oxo-1,3-diazaspiro[4.5]dek-1-en in Form eines hellbraunen Oels.

Beispiel 4: Eine Lösung von 0.59 g (1.6 mmol) 1-(2′-Cyanobiphenyl-4-ylmethyl)-4,4-diethyl-5-oxo-2-(n-propyl)-4,5-dihydro-1H-imidazol und 0.7 g (2.1 mmol) Tributylzinnazid in 20 ml Xylol-Gemisch wird 48 Stunden unter Rückfluss erhitzt, wobei nach 18 Stunden weitere 0.3 g Tributylzinnazid zugegeben werden. Nach Aufarbeitung analog Beispiel 1 unter Verwendung von 15 ml 2 N-Kalilauge und Reinigung durch Flash-Chromatographie erhält man 4,4-Diethyl-5-oxo-2-(n-propyl)-1-[2′-(1H-tetrazol-5-yl)biphenyl-4-ylmethyl]-4,5-dihydro-1H-imidazol als farblosen, amorphen Feststoff [Rf = 0.42 (Toluol/Isopropanol/Eisessig = 170/30/2)].

Das Ausgangsmaterial kann z.B. wie folgt hergestellt werden:

a) Analog Beispiel 3a) erhält man aus 2-Amino-2-ethyl-butansäure und Butanoylchlorid 2-Butanoylamino-2-ethyl-butansäure [Smp.: 161 - 162° (aus Wasser)].

b) Eine Lösung von 2. 15 g ( 10.7 mmol) 2-Butanoylamino-2-ethyl-butansäure und 2.0 g (16.7 mmol) N-Hydroxysuccinimid in 50 ml N,N-Dimethylformamid wird unter Rühren mit 2.6 g (12.5 mmol) N,N′-Dicyclohexylcarbodiimid versetzt. Nach 20 Minuten wird eine Lösung von 2.4 g (11 mmol) 4-Aminomethyl-2′-cyano-biphenyl in 20 ml N,N-Dimethylformamid zugegeben. Das Reaktionsgemisch wird 20 bis 24 Stunden gerührt und dann analog Beispiel 1 b) aufgearbeitet. Man erhält so 2-Butanoylamino-2-ethyl-butansäure-N-(2′-cyanobiphenyl-4-ylmethyl)amid als Oel [$R_f$ = 0.35 (Toluol/Isopropanol/-konzentriertes Ammoniak = 170/30/2)], das roh weiterverarbeitet werden kann.

c) Eine Lösung von 4.1 g (10.5 mmol) 2-Butanoylamino-2-ethyl-butansäure-N-(2′-cyanobiphenyl-4-ylmethyl)amid in 100 ml Xylol-Gemisch wird nach Zusatz von 2.0 g (10.5 mmol) p-Toluolsulfonsäure-monohydrat am Wasserabscheider unter Rückfluss erhitzt. Nach 6 Stunden wird analog Beispiel 3c) aufgearbeitet und das Rohprodukt an 400 g Silicagel flash-chromatographiert (Toluol/Methanol = 19/1). Die das Produkt enthaltenden Fraktionen ($R_f$ = 0.15) werden vereinigt und eingedampft und liefem so 1-(2′-Cyanobiphenyl-4-ylmethyl)-4,4-diethyl-5-oxo-2-(n-propyl)-4,5-dihydro-1H-imidazol in Form eines gelblichen Oels.

Beispiel 5: Eine Lösung von 0.33 g (1.0 mmol) 5-(n-Butyl)-3-oxo-4-(2′-cyanobiphenyl-4-ylmethyl)-2,3-dihydro-4H- 1,2,4-triazol und 0.43 g (1.3 mmol) Tributylzinnazid in 5 ml o-Xylol wird 28 Stunden unter Rückfluss erhitzt. Das Reaktionsgemisch wird mit 10 ml Toluol verdünnt, mit 4 ml 1 N-Natronlauge versetzt und 2 Stunden verrührt. Die wässrige Phase wird abgetrennt, mit Essigsäure auf pH 3 bis 4 gebracht und mit Ethylacetat extrahiert. Nach Trocknen und Abdampfen des Lösungsmittels erhält man ein Oel, aus dem allmählich 5-(n-Butyl)-3-oxo-4-[2′-(1H-tetrazol-5-yl)biphenyl-4-ylmethyl]-2,3-dihydro-4H-1,2,4-triazol auskristallisiert [Smp.: 213-215° (aus Acetonitril)].

Das Ausgangsmaterial kann beispielsweise wie folgt hergestellt werden:

a) Eine Lösung von 4.16 g (20 mmol) 4-Aminomethyl-2′-cyano-biphenyl und 7.0 ml 1,1,1-Trimethoxypentan in 40 ml Toluol wird 10 bis 12 Stunden unter Rückfluss erhitzt. Nach Eindampfen im Vakuum erhält man 1-[N-(2′-Cyanobiphenyl-4-ylmethyl)imino]-1-methoxy-pentan als Oel, das roh weiterverarbeitet wird.

b) 1.3 g (ca. 4 mmol) rohes 1-[N-(2′-Cyanobiphenyl-4-ylmethyl)imino]-1-methoxy-pentan und 0.5 g (5 mmol) Ethoxycarbonylhydrazin werden in 10 ml Toluol 20 Stunden unter Rückfluss erhitzt. Durch Waschen der abgekühlten Lösung, zuerst mit 5 ml wässriger 1 N-Methansulfonsäurelösung und anschliessend mit gesättigter Natriumhydrogencarbonatlösung, und Eindampfen der organischen Phase erhält man ein Oel, aus dem allmählich 5-(n-Butyl)-3-oxo-4-(2′-cyanobiphenyl-4-ylmethyl)-2,3-dihydro-4H-1,2,4-triazol auskristallisiert [Smp.: 134-135 ° (aus Acetonil)].

Beispiel 6: Eine Lösung von 3.7 g (10.3 mmol) 2-(n-Butyl)-1-(2′-cyanobiphenyl-4-ylmethyl)-4,4-dimethyl-5-oxo-4,5-dihydro-1H-imidazol und 4.4 g (13 mmol) Tributylzinnazid in 40 ml o-Xylol wird 26 Stunden unter Rückfluss erhitzt. Aufarbeitung analog Beispiel 1 ergibt ein Rohprodukt, aus dem durch Umkristallisation aus Ethylacetat 2-(n-Butyl)-4,4-dimethyl-5-oxo-1-[2′-(1H-tetrazol-5-yl)biphenyl-4-ylmethyl]-4,5-dihydro-1H-imidazol vom Smp. 180-181° erhalten wird.

Das Ausgangsmaterial kann beispielsweise wie folgt hergestellt werden:

Eine Lösung von 7.5 g (20 mmol) 2-Methyl-2-pentanoylamino-propansäure-N-(2′-cyanobiphenyl-4-ylmethyl)amid und 4.0 g (20 mmol) p-Toluolsulfonsäure in 200 ml Toluol wird 12 Stunden am Wasserabscheider zum Rückfluss erhitzt. Anschliessend wird das Reaktionsgemisch mit 20 ml 2 N-Natronlauge verrührt und die Toluolphase abgetrennt, mit Wasser gewaschen, getrocknet und im Vakuum eingedampft. Das so erhaltene Rohprodukt wird an Kieselgel flash-chromatographiert (Toluol/Methanol = 19/1). Die das Produkt enthaltenden Fraktionen ($R_f$ = 0.22) werden vereinigt und eingedampft und liefern so 2-(n-Butyl)-1-(2′-cyanobiphenyl-4-ylmethyl)-4,4-dimethyl-5-oxo-4,5-dihydro-1H-imidazol in Form eines farblosen Oels.

Beispiel 7: Zu einer Lösung von 2-(n-Butyl)-4,4-dimethyl-6-oxo-1-[2′-(1-triphenyl-methyl-1H-tetrazol-5-yl)biphenyl-4-ylmethyl]- 1,4,5,6-tetrahydro-pyrimidin (2.90 g, 4.4 mmol) in 10 ml $CH_2Cl_2$ werden bei Raumtemperatur 4.4 ml ethanolische Hydrogen-chloridlösung (10 N) zugegeben. Nach 30 Minuten wird das Reaktionsgemisch im Vakuum eingedampft, der Rückstand mit Toluol versetzt und das Gemisch erneut im Vakuum eingedampft. Nach Zugabe von Essigsäureethylester wird das Gemisch filtriert und der Filterkuchen aus Acetonitril umkristallisiert. Nach Trocknen im Vakuum bei 100° verbleibt 2-(n-Butyl)-4,4-dimethyl-6-oxo-1-[2′-(1H-tetrazol-5-yl)biphenyl-4-ylmethyl]-1,4,5,6-tetrahydro-pyrimidin-hydrochlorid [Smp.: 245° (Zersetzung)].

Das Ausgangsmaterial kann beispielsweise wie folgt hergestellt werden:

a) 3,3-Dimethylacrylsäureethylester (5.06 ml, 36.44 mmol) und Valeramidin (3.65 g, 36.44 mmol) werden 12 Stunden bei Raumtemperatur gerührt. Nach Eindampfen im Vakuum wird der Rückstand in 20 ml Ethanol gelöst und die Lösung mit 6 ml ethanolischer Hydrogenchloridlösung (10 N) versetzt. Der Niederschlag wird abfiltriert und der Filterkuchen aus Ethanol umkristallisiert. Man erhält so 2- (n-Butyl)-4,4-dimethyl-6-oxo-1,4,5,6-tetrahydro-pyrimidin-hydrochlorid (Smp.: 215-218°).

b) Zu einer Lösung von 2-(n-Butyl)-4,4-dimethyl-6-oxo-1,4,5,6-tetrahydro-pyrimidin-hydrochlorid ( 1.30 g, 5.94 mmol) in 20 ml N,N-Dimethylformamid wird NaH (80 % in Weissöl; 0.36 g, 11.9 mmol) zugegeben. Die Suspension wird 1 Stunde bei Raumtemperatur gerührt. Dann wird eine Lösung von 2′-(1-Triphenylmethyl-1H-tetrazol-5-yl)-biphenyl-4-ylmethylbromid (3.31 g, 5.94 mmol) in 25 ml N,N-Dimethylformamid zugetropft und das Reaktionsgemisch 12 Stunden bei Raumtemperatur gerührt. Nach Eindampfen im Vakuum wird der Rückstand zwischen Wasser und Essigsäureethylester verteilt und die organische Phase über $Na_2SO_4$ getrocknet und im Vakuum eingedampft. Flash-Chromatographie (Kieselgel 60, 40-63 μm, $CH_2Cl_2/CH_3OH$ = 95/5) ergibt 2-(n-Butyl)-4,4-dimethyl-6-oxo-1-[2′-(1-triphenylmethyl-1H-tetrazol-5-yl)biphenyl-4-yl-methyl]- 1,4,5,6-tetrahydro-pyrimidin, welches direkt weiterverarbeitet wird.

Beispiel 8: Ausgehend von 2-(n-Butyl)-5,5-dimethyl-6-oxo-1-[2′-(1-triphenylmethyl-1H-tetrazol-5-yl)biphenyl-4-ylmethyl]-1,4,5,6-tetrahydro-pyrimidin erhält man in der in Beispiel 7 beschriebenen Weise 2-(n-Butyl)-5,5-dimethyl-6-oxo-1-[2′-(1H-tetrazol-5-yl)-biphenyl-4-ylmethyl]-1,4,5,6-tetrahydro-pyrimidin-hydrochlorid [Smp.: 187-191° (Zersetzung)].

Das Ausgangsmaterial kann beispielsweise wie folgt hergestellt werden:

a) 3-Amino-2,2-dimethyl-propansäureethylester ( 1.45 g, 10 mmol) und Valeramidin (1.0 g, 10 mmol) werden 6 Stunden unter Rückfluss gerührt. Nach dem Eindampfen wird der Rückstand zwischen Wasser und Essigsäureethylester verteilt und die organische Phase über $Na_2SO_4$ getrocknet und im Vakuum eingedampft. Flash-Chromatographie (Kieselgel 60, 40-63 μm, Essigsäureethylester) ergibt 2-(n-Butyl)-5,5-dimethyl-6-oxo-1,4,5,6-tetrahydro-pyrimidin, welches durch Behandlung mit etherischer Hydrogen-chloridlösung in das Hydrochlorid überführt und in dieser Form direkt weiterverarbeitet wird.

b) Alkylierung von 2-(n-Butyl)-5,5-dimethyl-6-oxo-1,4,5,6-tetrahydro-pyrimidin-hydrochlorid mit 2′-(1-Triphenylmethyl-1H-tetrazol-5-yl)biphenyl-4-ylmethylbromid und anschliessende Flash-Chromatographie in der in Beispiel 7b) beschriebenen Weise ergibt 2-(n-Butyl)-5,5-dimethyl-6-oxo-1-[2′-(1-triphenylmethyl-1H-tetrazol-5-yl)biphenyl-4-yl-methyl]- 1,4,5,6-tetrahydro-pyrimidin, welches direkt weiterverarbeitet wird.

Beispiel 9: Eine Lösung von 5.0 g (13 mmol) 2-(n-Butyl)-3-(2′-cyanobiphenyl-4-ylmethyl)-4-oxo- 1,3-diazaspiro[4.4]non-1-en und 5.6 g (1 6.9 mmol) Tributylzinnazid in 100 ml o-Xylol wird 40 Stunden unter Rückfluss erhitzt. Aufarbeitung analog Beispiel 1 liefert ein teilkristallines Rohprodukt, dessen kristalliner Anteil nach Umkristallisation aus Isopropanol das reine 2-(n-Butyl)-4-oxo-3-[2′-(1H-tetrazol-5-yl)biphenyl-4-ylmethyl]-1,3-diazaspiro[4.4]non-1-en (Smp.: 181-183°) ergibt.

Das Ausgangsmaterial kann beispielsweise wie folgt hergestellt werden:

a) 1-Amino-1-benzyloxycarbonyl-cyclopentan wird mit Pentanoylchlorid zu 1-Benzyloxycarbonyl-1-pentanoylamino-cyclopentan (Smp.: 46-48°) umgesetzt.

b) Katalytische Debenzylierung von 1-Benzyloxycarbonyl-1-pentanoylamino-cyclopentan mittels Pd/C

(10%) in Methanol ergibt 1-Carboxy-1-pentanoylamino-cyclopentan (Smp.: 192- 194°).

c) Analog Beispiel 3b) erhält man unter Verwendung von 4.26 g (20 mmol) 1-Carboxy-1-pentanoylamino-cyclopentan das 1-(2′-Cyanobiphenyl-4-ylmethylaminocarbonyl)-1-pentanoylamino-cyclopentan [Smp.: 140- 142° (aus Ethylacetat)].

d) Eine Lösung von 7.3 g (18 mmol) 1-(2′-Cyanobiphenyl-4-ylmethylaminocarbonyl)-1 -pentanoylamino-cyclopentan und 3.5 g (18.4 mmol) p-Toluolsulfonsäuremonohydrat in 150 ml Toluol wird 20 Stunden am Wasserabscheider unter Rückfluss erhitzt. Aufarbeitung analog Beispiel 3c) ergibt das rohe 2-(n-Butyl)-3-(2′-cyanobiphenyl-4-ylmethyl)-4-oxo-1,3-diazaspiro[4.4]non-1-en in Form eines gelblichen Oels [$R_f$ = 0.61 (Toluol/Isopropanol/konzentriertes Ammoniak = 170/30/2)], das ohne zusätzliche Reinigung weiterumgesetzt wird.

Beispiel 10: Eine Lösung von 0.95 g (2.4 mmol) 2-(n-Butyl)-1-(2′-cyanobiphenyl-4-ylmethyl)-4,4-diethyl-5-oxo-4,5-dihydro-1H-imidazol und 1.22 g (3.6 mmol) Tributylzinnazid in 10 ml o-Xylol wird 48 Stunden unter Rückfluss erhitzt. Aufarbeitung analog Beispiel 1 ergibt ein Rohprodukt, aus dem durch Umkristallisation aus Ethylacetat/Diethylether 2-(n-Butyl)-4,4-diethyl-5-oxo-1-[2′-(1H-tetrazol-5-yl)biphenyl-4-ylmethyl]-4,5-dihydro-1H-imidazol (Smp.: 120-121°) erhalten wird.

Das Ausgangsmaterial kann beispielsweise wie folgt hergestellt werden:

a) Analog Beispiel 3a) erhält man aus 2-Amino-2-ethyl-butansäure und Pentanoylchlorid die 2-Ethyl-2-pentanoylamino-butansäure in Form eines Oels [$R_f$ = 0.27 (Toluol/Methanol = 4/1 )], das ohne zusätzliche Reinigung weiterumgesetzt wird.

b) Analog Beispiel 4b) erhält man aus 2-Ethyl-2-pentanoylamino-butansäure das 2-Ethyl-2-pentanoylamino-butansäure-N-(2′-cyanobiphenyl-4-ylmethyl)amid als Oel [$R_f$ = 0.46 (Toluol/Isopropanol/konzentriertes Ammoniak = 170/30/2)], das mittels Flash-Chromatographie (Toluol/Methanol = 40/1 ) gereinigt wird.

c) Eine Lösung von 1.0 g (2.46 mmol) 2-Ethyl-2-pentanoylamino-butansäure-N-(2′-cyanobiphenyl-4-ylmethyl)amid und 0.52 g (2.7 mmol) p-Toluolsulfonsäuremonohydrat in 50 ml o-Xylol wird 48 Stunden am Wasserabscheider unter Rückfluss erhitzt. Das abgekühlte Reaktionsgemisch wird mit 50 ml Ethylacetat verdünnt und mit 10 ml 2N Natronlauge alkalisch gestellt. Die organische Phase wird nacheinander mit Wasser und Sole gewaschen, getrocknet und im Vakuum eingedampft. Das in Form eines Oels anfallende 2-(n-Butyl)-1-(2′-cyanobiphenyl-4-ylmethyl)-4,4-diethyl-5-oxo-4,5-dihydro-1H-imidazol [$R_f$ = 0.45 (Toluol/Methanol = 4/1)] wird ohne zusätzliche Reinigung weiterumgesetzt.

Beispiel 11: In analoger Weise wie in einem der vorstehenden Beispiele beschrieben kann man auch herstellen:

1. 2-(n-Butyl)-6-oxo-1-[2′-(1H-tetrazol-5-yl)biphenyl-4-ylmethyl]-1,4,5,6-tetrahydropyrimin,
2. 2-(n-Butyl)-6-oxo-4,4,5,5-tetramethyl-1-[2′-(1H-tetrazol-5-yl)biphenyl-4-ylmethyl]-1,4,5,6-tetrahydro-pyrimidin,
3. 2-(n-Butyl)-4-isopropyl-5-oxo-1-[2′-(1H-tetrazol-5-yl)biphenyl-4-ylmethyl]-4,5-dihydro-1H-imidazol,
4. 1,3-Di-(n-butyl)-5-oxo-4-[2′-(1H-tetrazol-5-yl)biphenyl-4-ylmethyl]-1,5-dihydro-4H- 1,2,4-triazol,
5. 4,4-Dimethyl-5-oxo-2-(n-propyl)-1-[2′-(1H-tetrazol-5-yl)biphenyl-4-ylmethyl]-4,5-dihydro- 1H-imidazol,
6. 4,4-Dimethyl-5-oxo-2-(n-pentyl)-1-[2′-(1H-tetrazol-5-yl)biphenyl-4-ylmethyl]-4,5-dihydro-1H-imidazol,
7. 2-(n-Butyl)-4-isopropyl-4-methyl-5-oxo-1-[2′-(1H-tetrazol-5-yl)biphenyl-4-ylmethyl]-4,5-dihydro-1H-imidazol,
8. 3-(n-Butyl)-1-methyl-5-oxo-4-[2′-(1H-tetrazol-5-yl)biphenyl-4-ylmethyl]-1,5-dihydro-4H- 1,2,4-triazol,
9. 2-(n-Butyl)-4-methyl-5-oxo-1-[2′-(1H-tetrazol-5-yl)biphenyl-4-ylmethyl]-4,5-dihydro-1H-imidazol,
10. 2-(n-Butyl)-4-ethyl-5-oxo-1-[2′-(1H-tetrazol-5-yl)biphenyl-4-ylmethyl]-4,5-dihydro-1H-imidazol und
11. 2-(n-Butyl)-5-oxo-1-[2′-(1H-tetrazol-5-yl)biphenyl-4-ylmethyl]-4,5-dihydro-1H-imidazol,

jeweils in freier Form oder in Salzform, z. B. in Hydrochloridform.

Beispiel 12: Tabletten, enthaltend je 50 mg Wirkstoff, z.B. 2-(n-Butyl)-4,4-dimethyl-5-oxo-1-[2′-(1H-tetrazol-5-yl)biphenyl-4-ylmethyl]-4,5-dihydro-1H-imidazol, können wie folgt hergestellt werden:

Zusammensetzung (für 10000 Tabletten):

| | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q. s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesium-stearat und das hochdisperse Siliciumdioxid zu und presst das

Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 13: Lacktabletten, enthaltend je 100 mg Wirkstoff, z.B. 2-(n-Butyl)-4,4-dimethyl-5-oxo-1-[2'-(1H-tetrazol-5-yl)biphenyl-4-ylmethyl]-4,5-dihydro-1H-imidazol, können wie folgt hergestellt werden:

Zusammensetzung (für 1000 Tabletten):

| | |
|---|---|
| Wirkstoff | 100,00 g |
| Lactose | 100,0 g |
| Maisstärke | 70,00 g |
| Talk | 8,50 g |
| Calciumstearat | 1,50 g |
| Hydroxypropylmethylcellulose | 2,36 g |
| Schellack | 0,64 g |
| Wasser | q. s. |
| Dichlormethan | q. s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Dichlormethan lackiert (Endgewicht der Lack-tablette: 283 mg).

Beispiel 14: In analoger Weise wie in den Beispielen 12 und 13 beschrieben können auch Tabletten und Lacktabletten, enthaltend eine andere Verbindung I oder ein Tautomeres einer Verbindung I oder ein pharmazeutisch verwendbares Salz einer Verbindung I oder eines Tautomeren einer Verbindung I, z.B. gemäss einem der Beispiele 1 bis 11, hergestellt werden.

## Patentansprüche

1. Eine Verbindung der Formel

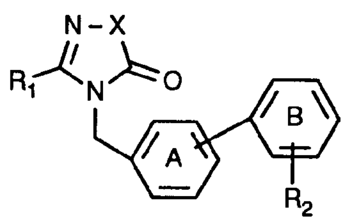

(I),

worin X die Gruppe der Formel $-C(R_3)R_4-[C(R_5)R_6]_p-[C(R_7)R_8]_q-$ (Ia) oder die Gruppe der Formel $-N(R_9)-$ (Ib) ist, $R_1$ unsubstituiertes oder ein- oder mehrfach substituiertes Niedeialkyl, Niederalkenyl oder Niederalkinyl, wobei die Substituenten aus der Gruppe ausgewählt sein können, die aus Hydroxy und Halogen besteht, Cycloakyl, Cycloalkenyl, Phenylniederalkyl, Phenylniederalkenyl oder Phenylniederalkinyl ist, $R_2$ Carboxy, 1H-Tetrazol-5-yl, $SO_3H$, $PO_2H_2$, $PO_3H_2$ oder Halogenalkansulfonylamino ist, entweder $R_3$, $R_4$, $R_5$, $R_6$, $R_7$ und $R_8$ unabhängig voneinander Wasserstoff, unsubstituiertes oder ein-oder mehrfach substituiertes Niederalkyl, Niederalkenyl oder Niederalkinyl, wobei die Substituenten aus der Gruppe ausgewählt sein können, die aus Halogen, gegebenenfalls verethertem Hydroxy, gegebenenfalls verestertem oder amidiertem Carboxy und gegebenenfalls substituiertem Amino besteht, gegebenenfalls verestertes oder amidiertes Carboxy, Cycloalkyl, Cycloalkenyl, einen durch O unterbrochenen aliphatischen Kohlenwasserstoffrest oder einen aromatischen Rest darstellen oder eines der drei Variablenpaare $R_3/R_4$, $R_5/R_6$ und $R_7/R_8$ einen zweiwertigen aliphatischen Kohlenwasserstoffrest darstellt und die Variablen der beiden anderen Variablenpaare unabhängig voneinander die vorstehend angegebenen Bedeutungen haben, $R_9$ Wasserstoff oder unsubstituiertes oder ein- oder mehrfach substituiertes Niederalkyl, Niederalkenyl oder Niederalkinyl, wobei die Substituenten aus der Gruppe ausgewählt sein können, die aus gegebenenfalls verethertem Hydroxy und gegebenenfalls verestertem oder amidiertem Carboxy besteht, ist, die Indices p und q unabhängig voneinander für 0 oder 1 stehen und die Ringe A und B unabhängig voneinander gegebenenfalls substituiert sind, oder gegebenenfalls ein Tautomeres davon, jeweils in freier Form oder in Salzform.

2. Eine Verbindung gemäss Anspruch 1 der Formel I, worin X die Gruppe Ia oder die Gruppe Ib ist, $R_1$ unsub-

stituiertes oder ein- oder mehrfach substituiertes Niederalkyl, Niederalkenyl oder Niederakinyl, wobei die Substituenten aus der Gruppe ausgewählt sein können, die aus Hydroxy und Halogen besteht, Cycloakyl, Cycloakenyl, Phenylniederalkyl, Phenylniederalkenyl oder Phenylniederalkinyl ist, $R_2$ Carboxy, 1H-Tetrazol-5-yl, $SO_3H$, $PO_2H_2$, $PO_3H_2$ oder Halogenalkansulfonylamino ist, entweder $R_3$, $R_4$, $R_5$, $R_6$, $R_7$ und $R_8$ unabhängig voneinander Wasserstoff, unsubstituiertes oder ein-oder mehrfach substituiertes Niederalkyl, Niederalkenyl oder Niederalkinyl, wobei die Substituenten aus der Gruppe ausgewählt sein können, die aus Halogen, Hydroxy, Niederalkoxy, Niederalkenyloxy, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl, Hydroxyniederalkyl, Niederalkoxyniederalkyl oder Phenyl-niederalkyl, -niederalkenyl oder -niederalkinyl unabhängig voneinander mono-oder disubstituiert oder durch Niederalkylen oder Niederalkylenoxyniederalkylen disubstituiert ist, Amino und durch Niederalkyl, Niederakenyl, Niederalkinyl oder Phenyl-niederalkyl, -niederalkenyl oder -niederalkinyl unabhängig voneinander mono-oder disubstituiertem oder durch Niederalkylen oder Niederalkylenoxyniederalkylen disubstituiertem Amino besteht, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl, Hydroxyniederalkyl, Niederalkoxyniederalkyl oder Phenyl-niederalkyl, -niederalkenyl oder -niederalkinyl unabhängig voneinander mono- oder disubstituiert oder durch Niederalkylen oder Niederalkylenoxyniederalkylen disubstituiert ist, Cycloalkyl, Cycloalkenyl, Niederalkoxy-niederalkyl, -niederalkenyl oder -niederalkinyl, Niederalkenyloxy-niederalkyl, -niederalkenyl oder -niederalkinyl, Phenyl, Naphthyl, Pyrrolyl, Pyrazolyl, Imidazolyl, Triazolyl, Tetrazolyl, Furyl, Thienyl oder Pyridyl darstellen oder eines der drei Variablenpaare $R_3/R_4$, $R_5/R_8$ und $R_7/R_8$ Niederalkylen oder Niederalkenylen darstellt und die Variablen der beiden anderen Variablenpaare unabhängig voneinander die unmittelbar vorstehend angegebenen Bedeutungen haben, $R_9$ Wasserstoff oder unsubstituiertes oder ein- oder mehrfach substituiertes Niederalkyl, Niederalkenyl oder Niederalkinyl, wobei die Substituenten aus der Gruppe ausgewählt sein können, die aus Niederalkoxy, Niederalkenyloxy, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, und Carbamoyl, dessen Aminogruppe unsubstituiert oder durch Niederalkyl, Niederalkenyl, Niederalkinyl, Hydroxyniederalkyl, Niederalkoxyniederalkyl oder Phenyl-niederalkyl, -niederalkenyl oder -niederalkinyl unabhängig voneinander mono- oder disubstituiert oder durch Niederalkylen oder Niederalkylenoxyniederalkylen disubstituiert ist, besteht, ist, die Indices p und q unabhängig voneinander für 0 oder 1 stehen und die Ringe A und B unabhängig voneinander unsubstituiert oder ein- oder mehrfach substituiert sind, wobei die Substituenten aus der Gruppe ausgewählt sein können, die aus Halogen, Hydroxy, Niederalkoxy, Niederalkenyloxy, Amino, durch Niederalkyl, Niederalkenyl, Niederalkinyl oder Phenyl-niederalkyl, -niederalkenyl oder -niederalkinyl unabhängig voneinander mono- oder disubstituiertem oder durch Niederalkylen oder Niederalkylenoxyniederakylen disubstituiertem Amino, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl, Hydroxyniederalkyl, Niederalkoxyniederalkyl oder Phenyl-niederalkyl, -niederalkenyl oder -niederalkinyl unabhängig voneinander mono- oder disubstituiert oder durch Niederakylen oder Niederalkylenoxyniederalkylen disubstituiert ist, Niederakyl, Niederalkenyl, Niederalkinyl, Niederalkoxy-niederalkyl, -niederalkenyl und -niederalkinyl, Niederalkenyloxy-niederalkyl, -niederalkenyl und -niederalkinyl und Trifluormethyl besteht, oder gegebenenfalls ein Tautomeres davon, jeweils in freier Form oder in Salzform.

3. Eine Verbindung gemäss Anspruch 1 der Formel I, worin X die Gruppe Ia oder die Gruppe Ib ist, $R_1$ Niederalkyl, Niederalkenyl, Niederalkinyl, Cycloalkyl oder Phenylniederalkyl ist, $R_2$ Carboxy, 1H-Tetrazol-5-yl, $SO_3H$, $PO_2H_2$, $PO_3H_2$ oder Halogenalkansulfonylamino ist, entweder $R_3$, $R_4$, $R_5$, $R_8$, $R_8$ und $R_9$ unabhängig voneinander Wasserstoff, Niederalkyl, Niederalkenyl, Niederalkinyl, Carboxy, Carboxy, welches durch einen Alkohol verestert ist, dessen alkoholische Hydroxyfunktion an Niederalkyl gebunden ist, Carbamoyl, dessen Aminogruppe unsubstituiert oder durch Niederalkyl unabhängig voneinander mono- oder disubstituiert ist, Cycloalkyl, Niederalkoxy-niederalkyl, Phenyl, Naphthyl, Pyrrolyl, Pyrazolyl, Imidazolyl, Triazolyl, Tetrazolyl, Furyl, Thienyl oder Pyridyl darstellen oder eines der drei Variablenpaare $R_3/R_4$, $R_5/R_8$ und $R_7/R_8$ Niederalkylen oder Niederalkenylen darstellt und die Variablen der beiden anderen Variablenpaare unabhängig voneinander die unmittelbar vorstehend angegebenen Bedeutungen haben, $R_9$ Wasserstoff, Niederalkyl, Niederalkenyl oder Niederalkinyl ist, entweder die Indices p und q für 0 stehen oder einer der

Indices p und q 0 ist und der andere der Indices p und q 1 ist, die Ringe A und B unabhängig voneinander unsubstituiert oder ein- oder mehrfach substituiert sind, wobei die Substituenten aus der Gruppe ausgewählt sein können, die aus Halogen, Hydroxy, Niederalkoxy, Amino, durch Niederalkyl oder Phenylniederalkyl unabhängig voneinander mono- oder disubstituiertem Amino, Carboxy, Carboxy, welches durch einen Akohol verestert ist, dessen alkoholische Hydroxyfunktion an Niederalkyl gebunden ist, Carbamoyl, dessen Aminogruppe unsubstituiert oder durch Niederalkyl oder Phenylniederalkyl unabhängig voneinander mono- oder disubstituiert oder durch Niederalkylen oder Niederalkylenoxyniederalkylen disubstituiert ist, Niederalkyl und Trifluormethyl besteht, und der Ring B in 4-Position des Ringes A und $R_2$ in einer 2-Position des Ringes B gebunden ist, oder gegebenenfalls ein Tautomeres davon, jeweils in freier Form oder in Salzform.

4. Eine Verbindung gemäss Anspruch 1 der Formel I, worin X die Gruppe Ia oder die Gruppe Ib ist, $R_1$ $C_1$-$C_4$-Alkyl ist, $R_2$ Carboxy oder 1H-Tetrazol-5-yl ist, entweder $R_3$, $R_4$, $R_5$, $R_6$, $R_7$ und $R_5$ unabhängig voneinander Wasserstoff oder $C_1$-$C_4$-Alkyl darstellen oder eines der drei Variablenpaare $R_3/R_4$, $R_5/R_5$ und $R_7/R_5$ $C_2$-$C_5$-Alkylen darstellt und die Variablen der beiden anderen Variablenpaare unabhängig voneinander die unmittelbar vorstehend angegebenen Bedeutungen haben, $R_9$ Wasserstoff oder $C_1$-$C_4$-Alkyl ist, entweder die Indices p und q für 0 stehen oder einer der Indices p und q 0 ist und der andere der Indices p und q 1 ist, die Ringe A und B unsubstituiert sind und der Ring B in 4-Position des Ringes A und $R_2$ in einer 2-Position des Ringes B gebunden ist, oder gegebenenfalls ein Tautomeres davon, jeweils in freier Form oder in Salzform.

5. Eine Verbindung gemäss Anspruch 1 der Formel I, worin X die Gruppe Ia oder die Gruppe Ib ist, $R_1$ $C_1$-$C_4$-Alkyl ist, $R_2$ 1H-Tetrazol-5-yl ist, entweder $R_3$, $R_4$, $R_5$, $R_6$, $R_7$ und $R_5$ unabhängig voneinander Wasserstoff oder $C_1$-$C_4$-Alkyl darstellen oder eines der drei Variablenpaare $R_3/R_4$, $R_5/R_5$ und $R_7/R_5$ $C_2$-$C_5$-Alkylen darstellt und die Variablen der beiden anderen Variablenpaare Wasserstoff bedeuten, $R_9$ Wasserstoff ist, entweder die Indices p und q für 0 stehen oder einer der Indices p und q 0 ist und der andere der Indices p und q 1 ist, die Ringe A und B unsubstituiert sind und der Ring B in 4-Position des Ringes A und $R_2$ in einer 2-Position des Ringes B gebunden ist, oder gegebenenfalls ein Tautomeres davon, jeweils in freier Form oder in Salzform.

6. Eine Verbindung gemäss Anspruch 1 der Formel I, worin X die Gruppe Ia ist, $R_1$ $C_1$-$C_4$-Alkyl ist, $R_2$ 1H-Tetrazol-5-yl ist, entweder $R_3$, $R_4$, $R_5$, $R_6$, $R_7$ und $R_8$ unabhängig voneinander Wasserstoff oder $C_1$-$C_4$-Alkyl darstellen oder eines der drei Variablenpaare $R_3/R_4$, $R_5/R_5$ und $R_7/R_5$ $C_2$-$C_5$-Alkylen darstellt und die Variablen der beiden anderen Variablenpaare Wasserstoff bedeuten, entweder die Indices p und q für 0 stehen oder einer der Indices p und q 0 ist und der andere der Indices p und q 1 ist, die Ringe A und B unsubstituiert sind und der Ring B in 4-Position des Ringes A und $R_2$ in einer 2-Position des Ringes B gebunden ist, in freier Form oder in Salzform.

7. Eine Verbindung gemäss Anspruch 1 der Formel I, worin X die Gruppe Ia ist, $R_1$ $C_1$-$C_4$-Alkyl ist, $R_2$ 1H-Tetrazol-5-yl ist, $R_3$, $R_4$, $R_5$, $R_5$, $R_7$ und $R_8$ unabhängig voneinander Wasserstoff oder $C_1$-$C_4$-Alkyl darstellen, entweder die Indices p und q für 0 stehen oder einer der Indices p und q 0 ist und der andere der Indices p und q 1 ist, die Ringe A und B unsubstituiert sind und der Ring B in 4-Position des Ringes A und $R_2$ in einer 2-Position des Ringes B gebunden ist, in freier Form oder in Salzform.

8. Eine Verbindung gemäss Anspruch 1 ausgewählt aus der Gruppe bestehend aus:
2-(n-Butyl)-4,4-dimethyl-5-oxo-1-[2′-(1H-tetrazol-5-yl)biphenyl-4-ylmethyl]-4,5-dihydro-1H-imidazol oder ein Salz davon;
2-(n-Butyl)-4-oxo-3-[2′-(1H-tetrazol-5-yl)biphenyl-4-ylmethyl]-1,3-diazaspiro[4.5]-dek-1-en oder ein Salz davon;
4,4-Diethyl-5-oxo-2-(n-propyl)-1-[2′-(1H-tetrazol-5-yl)biphenyl-4-ylmethyl]-4,5-dihydro-1H-imidazol oder ein Salz davon;
5-(n-Butyl)-3-oxo-4-[2′-(1H-tetrazol-5-yl)biphenyl-4-ylmethyl]-2,3-dihydro-4H- 1,2,4-triazol oder ein Tautomeres und/oder Salz davon;
2-(n-Butyl)-4,4-dimethyl-6-oxo- 1-[2′-(1H-tetrazol-5-yl)biphenyl-4-ylmethyl]- 1,4,5,6-tetrahydro-pyrimidin oder ein Salz davon;
2-(n-Butyl)-5,5-dimethyl-6-oxo-1-[2′-(1H-tetrazol-5-yl)biphenyl-4-ylmethyl]-1,4,5,6-tetrahydro-pyrimidin oder ein Salz davon;
2-(n-Butyl)-6-oxo-1-[2′-(1H-tetrazol-5-yl)biphenyl-4-ylmethyl]-1,4,5,6-tetrahydropyrimidin oder ein Salz

davon;
2-(n-Butyl)-6-oxo-4,4,5,5-tetramethyl-1-[2'-(1H-tetrazol-5-yl)biphenyl-4-ylmethyl]-1,4,5,6-tetrahydro-pyrimidin oder ein Salz davon;
2-(n-Butyl)-4,4-diethyl-5-oxo-1-[2'-(1H-tetrazol-5-yl)biphenyl-4-ylmethyl]-4,5-dihydro-ihydro-1H-imidazol oder ein Salz davon;
2-(n-Butyl)-4-oxo-3-[2'-(1H-tetrazol-5-yl)biphenyl-4-ylmethyl]-1,3-diazaspiro[4.4]-non-1-en oder ein Salz davon;
2-(n-Butyl)-4-isopropyl-5-oxo- 1 -[2'-(1H-tetrazol-5-yl)biphenyl-4-ylmethyl]-4,5-dihydro-1H-imidazol oder ein Salz davon;
1,3-Di-(n-butyl)-5-oxo-4-[2'-(1H-tetrazol-5-yl)biphenyl-4-ylmethyl]-1,5-dihydro-4H-1,2,4-triazol oder ein Salz davon;
4,4-Dimethyl-5-oxo-2-(n-propyl)-1-[2'-(1H-tetrazol-5-yl)biphenyl-4-ylmethyl]-4,5-dihydro-1H-imidazol oder ein Salz davon;
4,4-Dimethyl-5-oxo-2-(n-pentyl)-1-[2'-(1H-tetrazol-5-yl)biphenyl-4-ylmethyl]-4,5-dihydro-1H-imidazol oder ein Salz davon;
2-(n-Butyl)-4-isopropyl-4-methyl-5-oxo-1-[2'-(1H-tetrazol-5-yl)biphenyl-4-ylmethyl]-4,5-dihydro-1H-imidazol oder ein Salz davon;
3-(n-Butyl)-1-methyl-5-oxo-4-[2'-(1H-tetrazol-5-yl)biphenyl-4-ylmethyl]-1,5-dihydro-4H-1,2,4-triazol oder ein Salz davon;
2-(n-Butyl)-4-methyl-5-oxo-1-[2'-(1H-tetrazol-5-yl)biphenyl-4-ylmethyl]-4,5-dihydro-1H-imidazol oder ein Salz davon;
2-(n-Butyl)-4-ethyl-5-oxo-1-[2'-(1H-tetrazol-5-yl)biphenyl-4-ylmethyl]-4,5-dihydro-1H-imidazol- oder ein Salz davon; und
2-(n-Butyl)-5-oxo-1-[2'-(1H-tetrazol-5-yl)biphenyl-4-ylmethyl]-4,5-dihydro-1H-imidazol oder ein Salz davon.

9. Eine Verbindung gemäss einem der Ansprüche 1 bis 8 oder gegebenenfalls ein Tautomeres davon, jeweils in freier Form oder in Form eines pharmazeutisch verwendbaren Salzes, zur Anwendung in einem Verfahren zur therapeutischen Behandlung des menschlichen oder tierischen Körpers.

10. Eine Verbindung gemäss einem der Ansprüche 1 bis 9 oder gegebenenfalls ein Tautomeres davon, jeweils in freier Form oder in Form eines pharmazeutisch verwendbaren Salzes, zur Anwendung als Antihypertensivum.

11. Ein pharmazeutisches Präparat, als Wirkstoff enthaltend eine Verbindung gemäss einem der Ansprüche 1 bis 10 oder gegebenenfalls ein Tautomeres davon, jeweils in freier Form oder in Form eines pharmazeutisch verwendbaren Salzes, gegebenenfalls neben üblichen pharmazeutischen Hilfsstoffen.

12. Verfahren zur Herstellung einer Verbindung gemäss einem der Ansprüche 1 bis 8 der Formel I oder gegebenenfalls eines Tautomeren davon, jeweils in freier Form oder in Salzform, dadurch gekennzeichnet, dass man

a) in einer Verbindung der Formel

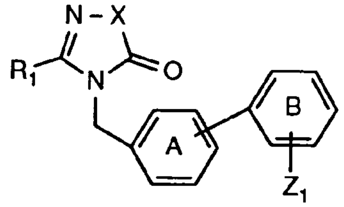

(II),

worin $Z_1$ einen in $R_2$ überführbaren Rest darstellt, oder in einem Salz davon $Z_1$ in $R_2$ überführt oder

b) eine Verbindung der Formel

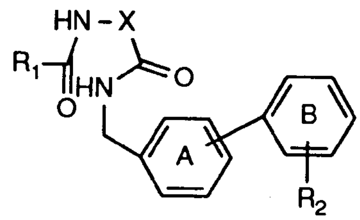

(III)

oder ein Salz davon cyclisiert oder
c) eine Verbindung der Formel

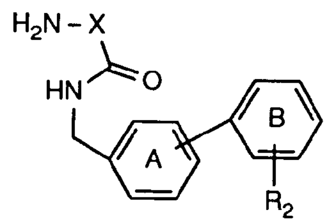

(IV)

oder ein Salz davon mit einer Verbindung der Formel

$R_1$-C($Z_2$($Z_3$)$Z_4$ (V),

worin entweder $Z_2$ und $Z_3$ gemeinsam gegebenenfalls funktionell abgewandeltes Oxo bedeuten und $Z_4$ eine nucleofuge Abgangsgruppe ist oder $Z_2$, $Z_3$ und $Z_4$ unabhängig voneinander für eine nucleofuge Abgangsgruppe stehen, umsetzt und jeweils, wenn erwünscht, eine verfahrensgemäss oder auf andere Weise erhältliche verbindung I oder ein Tautomeres davon, jeweils in freier Form oder in Salzform, in eine andere verbindung I oder ein Tautomeres davon überführt, ein verfahrensgemäss erhältliches Gemisch von Isomeren auftrennt und das gewünschte Isomere isoliert und/oder eine verfahrensgemäss erhältliche freie Verbindung I oder ein Tautomeres davon in ein Salz oder ein verfahrensgemäss erhältliches Salz einer Verbindung I oder eines Tautomeren davon in die freie Verbindung I oder ein Tautomeres davon oder in ein anderes Salz überführt.

**13.** Verwendung einer Verbindung gemäss einem der Ansprüche 1 bis 10 oder gegebenenfalls eines Tautomeren davon, jeweils in freier Form oder in Form eines pharmazeutisch verwendbaren Salzes, zur Herstellung eines pharmazeutischen Präparats.

**Patentansprüche für folgende Vertragsstaten: ES, GR**

**1.** Verfahren zur Herstellung einer Verbindung der Formel

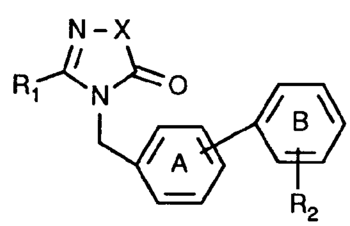

(I),

worin X die Gruppe der Formel -C($R_3$)$R_4$-[C($R_5$)$R_6$]$_p$-[C($R_7$)$R_8$]$_q$- (Ia) oder die Gruppe der Formel -N($R_9$)- (Ib) ist, $R_1$ unsubstituiertes oder ein- oder mehrfach substituiertes Niederakyl, Niederalkenyl oder Niederalkinyl, wobei die Substituenten aus der Gruppe ausgewählt sein können, die aus Hydroxy und Halogen besteht, Cycloakyl, Cycloalkenyl, Phenylniederalkyl, Phenylniederalkenyl oder Phenylniederalkinyl ist, $R_2$ Carboxy, 1H-Tetrazol-5-yl, $SO_3H$, $PO_2H_2$, $PO_3H_2$ oder Halogenalkansulfonylamino ist, entweder $R_3$, $R_4$, $R_5$, $R_6$, $R_7$ und $R_8$ unabhängig voneinander Wasserstoff, unsubstituiertes oder ein-oder mehrfach substituiertes Niederakyl, Niederalkenyl oder Niederalkinyl, wobei die Substituenten aus der Gruppe ausgewählt sein können, die aus Halogen, gegebenenfalls verethertem Hydroxy, gegebenenfalls verestertem oder amidiertem Carboxy und gegebenenfalls substituiertem Amino besteht, gegebenenfalls verestertes oder amidiertes Carboxy, Cycloalkyl, Cycloalkenyl, einen durch O unterbrochenen aliphatischen Kohlenwasserstoffrest oder einen aromatischen Rest darstellen oder eines der drei Variablenpaare $R_3/R_4$, $R_5/R_6$ und $R_7/R_5$ einen zweiwertigen aliphatischen Kohlenwasserstoffrest darstellt und die Variablen der beiden anderen Variablenpaare unabhängig voneinander die vorstehend angegebenen Bedeutungen haben, $R_9$ Wasserstoff oder unsubstituiertes oder ein- oder mehrfach substituiertes Niederalkyl, Niederalkenyl oder Niederalkinyl, wobei die Substituenten aus der Gruppe ausgewählt sein können, die aus gegebenenfalls verethertem Hydroxy und gegebenenfalls verestertem oder amidiertem Carboxy besteht, ist, die Indices p und q unabhängig voneinander für 0 oder 1 stehen und die Ringe A und B unabhängig voneinander gegebenenfalls substituiert sind, oder gegebenenfalls eines Tautomeren davon, jeweils in freier Form oder in Salzform, dadurch gekennzeichnet, dass man

a) in einer Verbindung der Formel

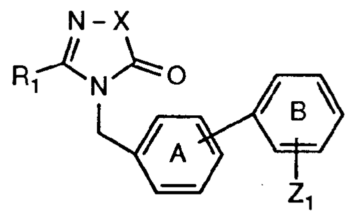

(II),

worin $Z_1$ einen in $R_2$ überführbaren Rest darstellt, oder in einem Salz davon $Z_1$ in $R_2$ überführt oder

b) eine Verbindung der Formel

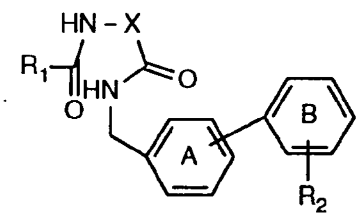

(III)

oder ein Salz davon cyclisiert oder

c) eine Verbindung der Formel

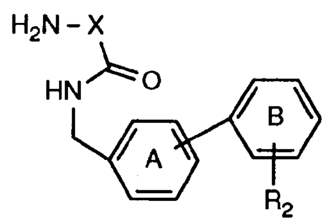

(IV)

oder ein Salz davon mit einer Verbindung der Formel

$$R_1\text{-}C(Z_2)(Z_3)Z_4 \quad (V),$$

worin entweder $Z_2$ und $Z_3$ gemeinsam gegebenenfalls funktionell abgewandeltes Oxo bedeuten und $Z_4$ eine nucleofuge Abgangsgruppe ist oder $Z_2$, $Z_3$ und $Z_4$ unabhängig voneinander für eine nucleofuge Abgangsgruppe stehen, umsetzt und jeweils, wenn erwünscht, eine verfahrensgemäss oder auf andere Weise erhältliche Verbindung I oder ein Tautomeres davon, jeweils in freier Form oder in Salzform, in eine andere Verbindung I oder ein Tautomeres davon überführt, ein verfahrensgemäss erhältliches Gemisch von Isomeren auftrennt und das gewünschte Isomere isoliert und/oder eine verfahrensgemäss erhältliche freie Verbindung I oder ein Tautomeres davon in ein Salz oder ein verfahrensgemäss erhältliches Salz einer Verbindung I oder eines Tautomeren davon in die freie Verbindung I oder ein Tautomeres davon oder in ein anderes Salz überführt.

**2.** Verfahren nach Anspruch 1 zur Herstellung einer Verbindung der Formel I, worin X die Gruppe Ia oder die Gruppe Ib ist, $R_1$ unsubstituiertes oder ein- oder mehrfach substituiertes Niederalkyl, Niederalkenyl oder Niederalkinyl, wobei die Substituenten aus der Gruppe ausgewählt sein können, die aus Hydroxy und Halogen besteht, Cycloalkyl, Cycloalkenyl, Phenylniederalkyl, Phenylniederalkenyl oder Phenylnie-

deralkinyl ist, $R_2$ Carboxy, 1H-Tetrazol-5-yl, $SO_3H$, $PO_2H_2$, $PO_3H_2$ oder Halogenalkansulfonylamino ist, entweder $R_3$, $R_4$, $R_5$, $R_6$, $R_7$ und $R_8$ unabhängig voneinander Wasserstoff, unsubstituiertes oder ein- oder mehrfach substituiertes Niederalkyl, Niederalkenyl oder Niederalkinyl, wobei die Substituenten aus der Gruppe ausgewählt sein können, die aus Halogen, Hydroxy, Niederalkoxy, Niederalkenyloxy, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl, Hydroxyniederalkyl, Niederalkoxyniederalkyl oder Phenyl-niederalkyl, -niederalkenyl oder -niederalkinyl unabhängig voneinander mono- oder disubstituiert oder durch Niederalkylen oder Niederalkylenoxyniederalkylen disubstituiert ist, Amino und durch Niederalkyl, Niederalkenyl, Niederalkinyl oder Phenyl-niederalkyl, -niederalkenyl oder -niederalkinyl unabhängig voneinander mono-oder disubstituiertem oder durch Niederalkylen oder Niederalkylenoxyniederalkylen disubstituiertem Amino besteht, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl, Hydroxyniederalkyl, Niederalkoxyniederalkyl oder Phenyl-niederalkyl, -niederalkenyl oder -niederalkinyl unabhängig voneinander mono- oder disubstituiert oder durch Niederalkylen oder Niederalkylenoxyniederalkylen disubstituiert ist, Cycloalkyl, Cycloalkenyl, Niederalkoxy-niederalkyl, -niederalkenyl oder -Niederalkinyl, Niederalkenyloxy-niederalkyl, -niederalkenyl oder -niederalkinyl, Phenyl, Naphthyl, Pyrrolyl, Pyrazolyl, Imidazolyl, Triazolyl, Tetrazolyl, Furyl, Thienyl oder Pyridyl darstellen oder eines der drei Variablenpaare $R_3/R_4$, $R_5/R_6$ und $R_7/R_8$ Niederalkylen oder Niederalkenylen darstellt und die Variablen der beiden anderen Variablenpaare unabhängig voneinander die unmittelbar vorstehend angegebenen Bedeutungen haben, $R_9$ Wasserstoff oder unsubstituiertes oder ein- oder mehrfach substituiertes Niederalkyl, Niederalkenyl oder Niederalkinyl, wobei die Substituenten aus der Gruppe ausgewählt sein können, die aus Niederalkoxy, Niederalkenyloxy, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, und Carbamoyl, dessen Aminogruppe unsubstituiert oder durch Niederalkyl, Niederalkenyl, Niederalkinyl, Hydroxyniederalkyl, Niederalkoxyniederalkyl oder Phenyl-niederalkyl, -niederalkenyl oder -niederalkinyl unabhängig voneinander mono- oder disubstituiert oder durch Niederalkylen oder Niederalkylenoxyniederalkylen disubstituiert ist, besteht, ist, die Indices p und q unabhängig voneinander für 0 oder 1 stehen und die Ringe A und B unabhängig voneinander unsubstituiert oder ein- oder mehrfach substituiert sind, wobei die Substituenten aus der Gruppe ausgewählt sein können, die aus Halogen, Hydroxy, Niederalkoxy, Niederalkenyloxy, Amino, durch Niederalkyl, Niederalkenyl, Niederalkinyl oder Phenyl-niederalkyl, -niederalkenyl oder -niederalkinyl unabhängig voneinander mono- oder disubstituiertem oder durch Niederalkylen oder Niederalkylenoxyniederalkylen disubstituiertem Amino, Carboxy, Carboxy, welches durch einen Alkohol verestert ist, dessen alkoholische Hydroxyfunktion an Niederalkyl, Niederalkenyl, Niederalkinyl oder Niederalkoxy-niederalkyl, -niederalkenyl oder -niederalkinyl gebunden ist, Carbamoyl, dessen Aminogruppe unsubstituiert oder durch Niederalkyl, Niederalkenyl, Niederalkinyl, Hydroxyniederalkyl, Niederalkoxyniederalkyl oder Phenyl-niederalkyl, -niederalkenyl oder -niederalkinyl unabhängig voneinander mono- oder disubstituiert oder durch Niederalkylen oder Niederalkylenoxyniederalkylen disubstituiert ist, Niederalkyl, Niederalkenyl, Niederalkinyl, Niederalkoxy-niederalkyl, -niederalkenyl und -niederalkinyl, Niederalkenyloxy-niederalkyl, -niederalkenyl und -niederalkinyl und Trifluormethyl besteht, oder gegebenenfalls eines Tautomeren davon, jeweils in freier Form oder in Salzform.

3. Verfahren nach Anspruch 1 zur Herstellung einer Verbindung der Formel I, worin X die Gruppe Ia oder die Gruppe Ib ist, $R_1$ Niederalkyl, Niederalkenyl, Niederalkinyl, Cycloalkyl oder Phenylniederalkyl ist, $R_2$ Carboxy, 1H-Tetrazol-5-yl, $SO_3H$, $PO_2H_2$, $PO_3H_2$ oder Halogenalkansulfonylamino ist, entweder $R_3$, $R_4$, $R_5$, $R_6$, $R_7$ und $R_8$ unabhängig voneinander Wasserstoff, Niederalkyl, Niederalkenyl, Niederalkinyl, Carboxy, Carboxy, welches durch einen Alkohol verestert ist, dessen alkoholische Hydroxyfunktion an Niederalkyl gebunden ist, Carbamoyl, dessen Aminogruppe unsubstituiert oder durch Niederalkyl unabhängig voneinander mono- oder disubstituiert ist, Cycloalkyl, Niederalkoxy-niederalkyl, Phenyl, Naphthyl, Pyrrolyl, Pyrazolyl, Imidazolyl, Triazolyl, Tetrazolyl, Furyl, Thienyl oder Pyridyl darstellen oder eines der drei Variablenpaare $R_3/R_4$, $R_5/R_8$ und $R_7/R_8$ Niederalkylen oder Niederalkenylen darstellt und die Variablen der beiden anderen Variablenpaare unabhängig voneinander die unmittelbar vorstehend angegebenen Bedeutungen haben, $R_9$ Wasserstoff, Niederalkyl, Niederalkenyl oder Niederalkinyl ist, entweder die Indices p und q für 0 stehen oder einer der Indices p und q 0 ist und der andere der Indices p und q 1 ist, die Ringe A und B unabhängig voneinander unsubstituiert oder ein- oder mehrfach substituiert sind, wobei

die Substituenten aus der Gruppe ausgewählt sein können, die aus Halogen, Hydroxy, Niederalkoxy, Amino, durch Niederalkyl oder Phenylniederalkyl unabhängig voneinander mono- oder disubstituiertem Amino, Carboxy, Carboxy, welches durch einen Alkohol verestert ist, dessen alkoholische Hydroxyfunktion an Niederalkyl gebunden ist, Carbamoyl, dessen Aminogruppe unsubstituiert oder durch Niederalkyl oder Phenylniederalkyl unabhängig voneinander mono- oder disubstituiert oder durch Niederalkylen oder Niederalkylenoxyniederalkylen disubstituiert ist, Niederalkyl und Trifluormethyl besteht, und der Ring B in 4-Position des Ringes A und $R_2$ in einer 2-Position des Ringes B gebunden ist, oder gegebenenfalls eines Tautomeren davon, jeweils in freier Form oder in Salzform.

4. Verfahren nach Anspruch 1 zur Herstellung einer Verbindung der Formel I, worin X die Gruppe Ia oder die Gruppe Ib ist, $R_1$ $C_1$-$C_4$-Alkyl ist, $R_2$ Carboxy oder 1H-Tetrazol-5-yl ist, entweder $R_3$, $R_4$, $R_5$, $R_6$, $R_7$ und $R_8$ unabhängig voneinander Wasserstoff oder $C_1$-$C_4$-Alkyl darstellen oder eines der drei Variablenpaare $R_3$/$R_4$, $R_5$/$R_6$ und $R_7$/$R_8$ $C_2$-$C_5$-Alkylen darstellt und die Variablen der beiden anderen Variablenpaare unabhängig voneinander die unmittelbar vorstehend angegebenen Bedeutungen haben, $R_9$ Wasserstoff oder $C_1$-$C_4$-Alkyl ist, entweder die Indices p und q für 0 stehen oder einer der Indices p und q 0 ist und der andere der Indices p und q 1 ist, die Ringe A und B unsubstituiert sind und der Ring B in 4-Position des Ringes A und $R_2$ in einer 2-Position des Ringes B gebunden ist, oder gegebenenfalls eines Tautomeren davon, jeweils in freier Form oder in Salzform.

5. Verfahren nach Anspruch 1 zur Herstellung einer Verbindung der Formel I, worin X die Gruppe Ia oder die Gruppe Ib ist, $R_1$ $C_1$-$C_4$-Alkyl ist, $R_2$ 1H-Tetrazol-5-yl ist, entweder $R_3$, $R_4$, $R_6$, $R_6$, $R_7$ und $R_8$ unabhängig voneinander Wasserstoff oder $C_1$-$C_4$-Alkyl darstellen oder eines der drei Variablenpaare $R_3$/$R_4$, $R_6$/$R_6$ und $R_7$/$R_6$ $C_2$-$C_5$-Alkylen darstellt und die Variablen der beiden anderen Variablenpaare Wasserstoff bedeuten, $R_9$ Wasserstoff ist, entweder die Indices p und q für 0 stehen oder einer der Indices p und q 0 ist und der andere der Indices p und q 1 ist, die Ringe A und B unsubstituiert sind und der Ring B in 4-Position des Ringes A und $R_2$ in einer 2-Position des Ringes B gebunden ist, oder gegebenenfalls eines Tautomeren davon, jeweils in freier Form oder in Salzform.

6. Verfahren nach Anspruch 1 zur Herstellung einer Verbindung der Formel I, worin X die Gruppe Ia ist, $R_1$ $C_1$-$C_4$-Alkyl ist, $R_2$ 1H-Tetrazol-5-yl ist, entweder $R_3$, $R_4$, $R_6$, $R_6$, $R_7$ and $R_8$ unabhängig voneinander Wasserstoff oder $C_1$-$C_4$-Alkyl darstellen oder eines der drei Variablenpaare $R_3$/$R_4$, $R_5$/$R_6$ und $R_7$/$R_6$ $C_2$-$C_5$-Alkylen darstellt und die Variablen der beiden anderen Variablenpaare Wasserstoff bedeuten, entweder die Indices p und q für 0 stehen oder einer der Indices p und q 0 ist und der andere der Indices p und q 1 ist, die Ringe A und B unsubstituiert sind und der Ring B in 4-Position des Ringes A und $R_2$ in einer 2-Position des Ringes B gebunden ist, in freier Form oder in Salzform.

7. Verfahren nach Anspruch 1 zur Herstellung einer Verbindung der Formel I, worin X die Gruppe Ia ist, $R_1$ $C_1$-$C_4$-Alkyl ist, $R_2$ 1H-Tetrazol-5-yl ist, $R_3$, $R_4$, $R_5$, $R_6$, $R_7$ und $R_8$ unabhängig voneinander Wasserstoff oder $C_1$-$C_4$-Alkyl darstellen, entweder die Indices p und q für 0 stehen oder einer der Indices p und q 0 ist und der andere der Indices p und q 1 ist, die Ringe A und B unsubstituiert sind und der Ring B in 4-Position des Ringes A und $R_2$ in einer 2-Position des Ringes B gebunden ist, in freier Form oder in Salzform.

8. Verfahren nach Anspruch 1 zur Herstellung einer Verbindung ausgewählt aus der Gruppe bestehend aus:
2-(n-Butyl)-4,4-dimethyl-5-oxo-1-[2′-(1H-tetrazol-5-yl)biphenyl-4-ylmethyl]-4,5-dihydro-1H-imidazol oder ein Salz davon;
2-(n-Butyl)-4-oxo-3-[2′-(1H-tetrazol-5-yl)biphenyl-4-ylmethyl]-1,3-diazaspiro[4.5]-dek-1-en oder ein Salz davon;
4,4-Diethyl-5-oxo-2-(n-propyl)-1-[2′-(1H-tetrazol-5-yl)biphenyl-4-ylmethyl]-4,5-dihydro-1H-imidazol oder ein Salz davon;
5-(n-Butyl)-3-oxo-4-[2′-(1H-tetrazol-5-yl)biphenyl-4-ylmethyl]-2,3-dihydro-4H- 1,2,4-triazol oder ein Tautomeres und/oder Salz davon;
2-(n-Butyl)-4,4-dimethyl-6-oxo-1-[2′-(1H-tetrazol-5-yl)biphenyl-4-ylmethyl] - 1,4,5,6-tetrahydro-pyrimidin oder ein Salz davon;
2-(n-Butyl)-5,5-dimethyl-6-oxo-1-[2′-(1H-tetrazol-5-yl)biphenyl-4-ylmethyl]-1,4,5,6-tetrahydro-pyrimidin oder ein Salz davon;
2-(n-Butyl)-6-oxo-1-[2′-(1H-tetrazol-5-yl)biphenyl-4-ylmethyl]-1,4,5,6-tetrahydro-pyrimidin oder ein Salz davon;
2-(n-Butyl)-6-oxo-4,4,5,5-tetramethyl-1-[2′-(1H-tetrazol-5-yl)biphenyl-4-ylmethyl]-1,4,5,6-tetrahydro-pyrimidin oder ein Salz davon;
2-(n-Butyl)-4,4-diethyl-5-oxo-1-[2′-(1H-tetrazol-5-yl)biphenyl-4-ylmethyl]-4,5-dihydro-1H-imidazol oder ein Salz davon;
2-(n-Butyl)-4-oxo-3-[2′-(1H-tetrazol-5-yl)biphenyl-4-ylmethyl]-1,3-diazaspiro[4.4]-non-1-en oder ein Salz davon;
2-(n-Butyl)-4-isopropyl-5-oxo-1-[2′-(1H-tetrazol-5-yl)biphenyl-4-ylmethyl]-4,5-dihydro-1H-imidazol oder ein Salz davon;
1,3-Di-(n-butyl)-5-oxo-4-[2′-(1H-tetrazol-5-yl)biphenyl-4-ylmethyl]-1,5-dihydro-4H-1,2,4-triazol oder ein Salz davon;
4,4-Dimethyl-5-oxo-2-(n-propyl)-1-[2′-(1H-tetrazol-5-yl)biphenyl-4-ylmethyl]4,5-dihydro-1H-imidazol oder ein Salz davon;
4,4-Dimethyl-5-oxo-2-(n-pentyl)-1-[2′-(1H-tetrazol-5-yl)biphenyl-4-ylmethyl]-4,5-dihydro-1H-imidazol oder ein Salz davon;
2-(n-Butyl)-4-isopropyl-4-methyl-5-oxo-1-[2′-(1H-tetrazol-5-yl)biphenyl-4-ylmethyl]-4,5-dihydro-1H-imid azol oder ein Salz davon;
3-(n-Butyl)-1-methyl-5-oxo-4-[2′-(1H-tetrazol-5-yl)biphenyl-4-ylmethyl]-1,5-dihydro-4H- 1,2,4-triazol oder ein Salz davon;
2-(n-Butyl)-4-methyl-5-oxo-1-[2′-(1H-tetrazol-5-yl)biphenyl-4-ylmethyl]-4,5-dihydro-1H-imidazol oder ein Salz davon;
2-(n-Butyl)-4-ethyl-5-oxo-1-[2′-(1H-tetrazol-5-yl)biphenyl-4-ylmethyl]-4,5-dihydro-1H-imidazol oder ein Salz davon; und
2-(n-Butyl)-5-oxo-1-[2′-(1H-tetrazol-5-yl)biphenyl-4-ylmethyl]-4,5-dihydro-1H-imidazol oder ein Salz davon.

**9.** Verfahren zur Herstellung eines pharmazeutischen Präparates enthaltend eine Verbindung der Formel I gemäss einem der Ansprüche 1-8, gegebenenfalls ein Tautomeres davon, jeweils in freier Form oder in Form eines pharmazeutisch verwendbaren Salzes, dadurch gekennzeichnet, dass man den Wirkstoff, gegebenenfalls unter Beimischung von üblichen pharmazeutischen Hilfsstoffen, zu einem pharmazeutischen Präparat verarbeitet.

Europäisches Patentamt

# EUROPÄISCHER RECHERCHENBERICHT

Nummer der Anmeldung

EP 91810697.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| P,X | EP - A - 0 412 594 (MERCK) * Ansprüche 1,9,10; Beispiele 1,2 * | 1,11 | C 07 D 249/12<br>C 07 D 233/70<br>C 07 D 239/22<br>C 07 D 243/04<br>A 61 K 31/415<br>A 61 K 31/41<br>A 61 K 31/505 |
| P,A | EP - A - 0 409 332 (MERCK) * Ansprüche 1,5 * | 1,11 | |
| A | EP - A - 0 323 841 (DU PONT) * Ansprüche 1,10,11 * | 1,11 | |

RECHERCHIERTE SACHGEBIETE (Int Cl 5)

C 07 D 249/00
C 07 D 233/00
C 07 D 239/00
C 07 D 243/00

Der vorliegende Recherchenbericht wurde für alle Patentansprüche erstellt.

| Recherchenort | Abschlußdatum der Recherche | Prüfer |
|---|---|---|
| WIEN | 02-12-1991 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82